# EUROPÄISCHE PATENTANMELDUNG

(11) **EP 3 056 189 A1**
(43) Veröffentlichungstag der Anmeldung: **17.08.2016**
(21) Anmeldenummer: 16163415.9
(22) Anmeldetag: 01.10.2008
(51) Int. Cl.: A61K 8/02, A61K 8/55, A61Q 19/00

(54) **TOPISCH ZU APPLIZIERENDE KOSMETISCHE ODER PHARMAZEUTISCHE ZUSAMMENSETZUNG**

(30) Priorität: 02.10.2007 DE 102007047304
(62) Teilanmeldung aus: 08801349.5
(71) Anmelder: Kuhs GmbH, 79541 Lörrach (DE)
(72) Erfinder: Albrecht, Martin, 51519 Odenthal (DE)
(74) Vertreter: Vossius & Partner Patentanwälte Rechtsanwälte mbB

(57) **Zusammenfassung**

Es wird eine topisch zu applizierende kosmetische oder pharmazeutische Zusammensetzung, die eine hydrophile äußere Phase, mindestens einen kosmetischen und/oder pharmazeutischen Wirkstoff sowie mindestens eine Trägersubstanz für den Wirkstoff aufweist, beschrieben. Hierbei bildet die Trägersubstanz solche Strukturen aus, die mindestens zwei, sandwichartig übereinander angeordnete lamellare Doppelmembranschichten umfaßt, wobei zwischen benachbarten parallel zueinander ausgerichteten Doppelmembranschichten jeweils eine Schicht einer inneren Phase angeordnet ist. Der Wirkstoff ist in der Doppelmembranschicht und in der Schicht der inneren Phase derart verteilt, daß die Schicht der inneren Phase den Wirkstoff in einem Konzentrationsbereich zwischen 2 Gew.% und 98 Gew.% und die Doppelmembranschicht den Wirkstoff in einer Konzentration zwischen 98 Gew.% und 2 Gew.%, jeweils bezogen auf die Gesamtkonzentration an Wirkstoff, enthält und die äußere Phase keinen oder nahezu keinen Wirkstoff aufweist.

## Beschreibung

Die vorliegende Erfindung betrifft eine topisch zu applizierende kosmetische oder pharmazeutische Zusammensetzung mit den Merkmalen des Oberbegriffs des Patentanspruchs 1.

Topisch zu applizierende kosmetische oder pharmazeutische Zusammensetzungen sind mit unterschiedlichen Inhaltsstoffen bekannt und auch weit verbreitet. Hier sind nur beispielsweise die typischen Öl-in-Wasser-Emulsionen oder Wasser-in-Öl-Emulsionen, die Gele, Salben oder Lotionen zu nennen, wobei gemeinsam für die zuvor genannten bekannten Zusammensetzungen ihre Aufgabe dahingehend zu formulieren ist, daß sie eine gewisse kosmetische oder pharmazeutische Wirksamkeit besitzen, die sich nach einer gewissen Zeit nach lokaler Anwendung einstellen muß.

Eine topisch zu applizierende kosmetische oder pharmazeutische Zusammensetzung, die eine hydrophile äußere Phase, mindestens einen kosmetischen und/oder pharmazeutischen Wirkstoff sowie mindestens eine Trägersubstanz für den Wirkstoff aufweist und bei der mindestens eine Trägersubstanz vorhanden ist, die solche Strukturen ausbildet, bei denen mindestens zwei, sandwichartig übereinander angeordnete lamellare Doppelmembranschichten vorhanden sind, wird erstmals in der DE 102 13 304 A angesprochen. Diese deutsche Patentanmeldung, die auf dieselbe Anmelderin wie die vorliegende Anmeldung zurückgeht, stellt jedoch schwerpunktmäßig heraus, daß die hydrophile Phase Wasser ist und daß desweiteren die bekannte Zusammensetzung als wesentliche Inhaltsstoffe Methylglycin, Dimethylglycin und/oder Methylmethionin enthalten muß. Wie der pharmazeutische Wirkstoff in der bekannten Zusammensetzung verteilt ist, läßt die DE 102 13 304 A offen. Eine derartige Aussage findet sich auch nicht in der DE 101 08 097 A, die ebenfalls auf die Anmelderin der vorliegenden Anmeldung zurückgeht, da sich diese, auf eine kosmetische Zusammensetzung gerichtete Anmeldung ebenfalls schwerpunktmäßig darauf bezieht, daß in der bekannten Zusammensetzung insbesondere die Inhaltsstoffe Methylglycin, Dimethylglycin und/oder Methylmethionin enthalten sein müssen.

Ergänzend zu diesem Stand der Technik wird auf die vorangemeldete, jedoch noch nicht offengelegte DE 10 2006 015 544 verwiesen, die ebenfalls auf die Anmelderin der vorliegenden Anmeldung zurückgeht, wobei in dieser Anmeldung topisch zu applizierende Zusammensetzungen zur Anwendung beim Säugling oder Kleinkind vorbeschrieben sind, derart, daß die dort beschriebenen bekannten Zusammensetzungen einen speziellen Wirkstoff, bei dem es sich um einen in einer hydrophilen Flüssigkeit schlecht löslichen, anti-entzündlichen Wirkstoff handelt. Dieser, in der hydrophilen Flüssigkeit schlecht lösliche anti-entzündliche Wirkstoff ist bei der vorangemeldeten und nachveröffentlichten DE 10 2006 015 544 in dem Material, das die lamellare Doppelmembranschicht ausbildet, homogen dispergiert, so daß dementsprechend die die Doppelmembranschicht umgebende hydrophile Flüssigkeit frei von anti-entzündlichen Wirkstoffen ist.

Der vorliegenden Erfindung liegt die Aufgabe zugrunde, eine topisch zu applizierende kosmetische oder pharmazeutische Zusammensetzung zur Verfügung zu stellen, die unter Berücksichtigung einer hohen Lagerstabilität eine besonders ausgeprägte kosmetische bzw. pharmazeutische Wirksamkeit besitzt.

Diese Aufgabe wird erfindungsgemäß durch eine topisch zu applizierende kosmetische oder pharmazeutische Zusammensetzung mit den kennzeichnenden Merkmalen des Patentanspruchs 1 gelöst.

Die erfindungsgemäße topisch zu applizierende kosmetische oder pharmazeutische Zusammensetzung, die nachfolgend auch kurz nur als erfindungsgemäße Zusammensetzung bezeichnet wird, weist eine hydrophile äußere Phase auf und enthält desweiteren mindestens einen kosmetischen und/oder pharmazeutischen Wirkstoff. Desweiteren umfaßt die erfindungsgemäße Zusammensetzung mindestens eine Trägersubstanz für den Wirkstoff, wobei die Trägersubstanz solche Strukturen ausbildet, die mindestens zwei, sandwichartig übereinander angeordnete lamellare Doppelmembranschichten besitzt. Zwischen benachbarten, parallel zueinander ausgerichteten Doppelmembranschichten ist bei der erfindungsgemäßen Zusammensetzung jeweils eine Schicht einer inneren Phase angeordnete. Im Unterschied zu der DE 10 2006 015 544 ist bei der erfindungsgemäßen Zusammensetzung der Wirkstoff in der Doppelmembranschicht und in der Schicht der inneren Phase verteilt, derart, daß die Schicht der inneren Phase den Wirkstoff in einem Konzentrationsbereich zwischen 2 Gew.% und 98 Gew.% und die Doppelmembranschicht den Wirkstoff in einer Konzentration zwischen 98 Gew.% und 2 Gew.%, jeweils bezogen auf die Gesamtkonzentration des in der erfindungsgemäßen Zusammensetzung vorhandenen Wirkstoffs, enthalten, während die äußere Phase, d.h. die Phase, die die spezielle Struktur von außen her umgibt, keinen oder nahezu keinen Wirkstoff enthält. Insbesondere bedeutet die zuvor wiedergegebene Aussage, wonach die erfindungsgemäße Zusammensetzung in der äußeren Phase keinen oder nahezu keinen Wirkstoff enthält, daß sich in dieser äußeren Phase, die insgesamt gesehen die spezielle lamellare Struktur umgibt und einbettet und die sich von der Schicht der inneren Phase, die ausschließlich zwischen benachbarten Doppelmembranschichten angeordnet ist, unterscheidet, insbesondere maximal 5 Gew.% des Wirkstoffes, bezogen auf die Gesamtkonzentration des Wirkstoffes in der erfindungsgemäßen Zusammensetzung, befindet.

Die erfindungsgemäße Zusammensetzung weist eine Reihe von Vorteilen auf. So ist zunächst festzuhalten, daß die erfindungsgemäße Zusammensetzung, bedingt durch ihre spezielle Struktur, wie diese zuvor beschrieben ist, schichtartig auf der Hautoberfläche angelagert und/oder schichtartig in die Interzellular-Lipidschicht eingelagert wird, so daß allein schon von daher ideale Voraussetzungen für eine optimale Penetration und damit auch, abhängig von dem jeweiligen Wirkstoff, eine schnelle Permeation des Wirkstoffes gegeben sind. Insbesondere ist die erfindungsgemäße Zusammensetzung geeignet, sich in Fehlstellen der Interzellular-Lipide aufgrund ihrer Strukturähnlichkeit mit den Interzellular-Lipiden einzulagern, so daß hier zwischen den Interzellular-Lipiden und der strukturähnlichen erfindungsgemäßen Zusammensetzung ein inniger Kontakt stattfindet, was wiederum die zuvor beschriebene Penetration und Permeation des Wirkstoffes beschleunigt und damit eine hohe kosmetische und/oder pharmazeutische Wirksamkeit begründet. Bedingt dadurch, daß innerhalb der erfindungsgemäßen Zusammensetzung der Wirkstoff sowohl in der lipophilen Doppelmembranschicht als auch in der zwischen benachbarten Doppelmembranschichten angeordneten Schicht der inneren Phase verteilt ist, findet während der Anwendung der erfindungsgemäßen Zusammensetzung innerhalb dieser speziellen Struktur eine ständige und dynamische neue Wiedereinstellung eines Gleichgewichtes dann statt, sobald der Wirkstoff aus der speziellen Struktur der erfindungsgemäßen Zusammensetzung in die Hautoberfläche penetriert. Dementsprechend findet ein ständig gleichbleibender Fluß an Wirkstoff aus der Zusammensetzung in die damit behandelten Bereiche der Haut statt, so daß aufgrund der gleichbleibenden Penetration die besonders hohe kosmetische und/oder pharmazeutische Wirksamkeit der erfindungsgemäßen Zusammensetzung erklärlich wird. Diese zuvor angesprochene gleichbleibende Penetration des Wirkstoffes wird für solche pharmazeutischen Wirkstoffe, die nicht nur lokal sondern auch systemisch wirken, als Ursache dafür angesehen, daß als Folge der gleichbleibenden Penetration auch eine gleichbleibende Permeation resultiert, so daß ein derartig in der zuvor beschriebenen Struktur der erfindungsgemäßen Zusammensetzung eingelagerte systemischer Wirkstoff über einen langen Zeitraum, so zum Beispiel zwischen zwei Stunden und 18 Stunden, in gleichbleibender Konzentration systemisch verfügbar ist. Aufgrund der zuvor bereits angesprochenen Ähnlichkeit der Struktur der erfindungsgemäßen Zusammensetzung mit der Struktur der Oberflächenfette und insbesondere mit der Struktur der Interzellular-Lipide ist die erfindungsgemäße Zusammensetzung in der Lage, Strukturdefekte in den Interzellular-Lipiden auszugleichen und dort wie eine Kittsubstanz zu wirken, so daß derartige Fehlstellen entsprechend mit Hilfe der erfindungsgemäßen Zusammensetzung ausgebessert werden und demnach die Haut wieder in eine gesunde Struktur versetzt, so daß sie die volle Schutzfunktion ausüben kann. Hierdurch wird verhindert, daß die Haut an diesen Fehlstellen in erhöhtem Maße austrocknet, daß Bakterien, Viren oder Allergene sich an den Fehlstellen einlagern und ggf. von dort in die tieferen Hautschichten penetrieren und somit als Ursache für entzündliche, oberflächlich ablaufende Hautirritationen oder Hautentzündungen dienen, wodurch insgesamt gesehen die hohe kosmetische Wirksamkeit der erfindungsgemäßen Zusammensetzung erklärbar wird. Ergänzend hierzu kann die erfindungsgemäßen Zusammensetzung nicht nur, wie zuvor beschrieben, zur Behandlung von geschädigter Haut verwendet werden, sondern die erfindungsgemäße Zusammensetzung läßt sich aufgrund ihrer speziellen Struktur auch im Vorfeld der eigentlichen Hautschädigung verwenden, d.h. somit als Schutzfunktion, so daß dementsprechend die Haut überhaupt nicht erst geschädigt wird. Ein derartiger Hautschutz, der insbesondere die gesunde Haut vor aggressiven äußerlichen Eingriffen, wie zum Beispiel extremer Lichtbestrahlung, aggressiven Medien, Salzwasser, Umweltnoxe oder Seifen, die aufgrund ihres Emulgatorgehaltes und ihrer häufigen Anwendung die Oberflächenlipide und insbesondere die Interzellular-Lipide schädigen, werden durch die erfindungsgemäße Zusammensetzung wirksam abgemildert, da die erfindungsgemäße Zusammensetzung aufgrund ihrer Struktur und der zuvor beschriebenen Verteilung der Wirkstoffe hervorragend mit der Oberflächenlipidschicht der Haut verträglich ist. Hierauf wird die hohe kosmetische Wirksamkeit der erfindungsgemäßen Zusammensetzung im Bereich des Hautschutzes zurückgeführt, zumal die erfindungsgemäße Zusammensetzung in der Lage ist, sowohl lipophile Wirkstoffe als auch lipophobe Wirkstoffe gleichzeitig und in den eingangs bei der erfindungsgemäßen Zusammensetzung quantifizierten Mengenverhältnisse innerhalb der lipophilen Doppelschicht und der inneren Phase einzulagern. Bedingt dadurch, daß bei der erfindungsgemäßen Zusammensetzung die äußere Phase keinen oder nahezu keinen Wirkstoff aufweist, besitzt die erfindungsgemäßen Zusammensetzung eine hohe Lagerstabilität, da hier der Wirkstoff zwischen der Doppelmembranschicht und der Schicht der inneren Phase verteilt und dort quasi eingekapselt ist, so daß dieser so eingelagerte Wirkstoff insbesondere gegenüber Alterungseinflüssen, hervorgerufen durch erhöhte Temperatur und/oder oxidativen Angriffe, wirksam geschützt ist. Die zuvor beschriebenen Vorteile der erfindungsgemäßen Zusammensetzung erklären nicht nur die hohe therapeutische sondern auch ebenso die hohe prophylaktische Wirkung der erfindungsgemäßen Zusammensetzung im kosmetischen und pharmazeutischen Bereich.

Um die zuvor beschriebene spezielle Struktur der erfindungsgemäßen Zusammensetzung experimentell nachzuweisen, besteht die Möglichkeit, hier als Meß- und Nachweismethode zunächst rasterelektronenmikroskopische Aufnahmen anzufertigen, die einen ersten Aufschluß über die Schichtstruktur erlauben. Hierzu werden insbesondere die zu untersuchenden Zusammensetzungen einer Gefrierbruchpräparation unterzogen. Hierauf erfolgt in der Regel eine Gefrierbruchätzung mit einer anschließenden Beschichtung durch Bedampfen mit einem Platin-Kohle-Substrat. Ein derartiges Präparat wird elektronenmikroskopisch betrachtet und abgebildet. Weitere detailliertere Auskünfte über die speziellen, zuvor beschriebenen und nachfolgend noch in der Zeichnung abgebildeten lamellaren Strukturen, die ein wesentliches Merkmal der erfindungsgemäßen Zusammensetzung sind, erlauben die isotherme Titrationskalorimetrie (ITC), die Infrarot-Spektroskopie und/oder die Differential-Scanning-Kalorimetrie (DSC), wie sie beispielsweise in "Bioelectrochemistry of Membranes, ed. by D. Walz, J. Teissié and G. Milazzo, 2004 Birkhäuser Verlag Basel/Schweiz" insbesondere "Chapter 3, Lipids" Autor: Alfred Blume, Seiten 61 bis 152 (mit weiteren Literaturnachweisen) und "Handbook of Thermal Analysis and Calorimetry, Vol 4: From Macromolecules to Man., R.B. Kemp, 1999 Elsevier Press B.V., Amsterdam, pp 109-173" (mit weiteren Liternaturnachweisen) ausführlich beschrieben sind.

Zuvor ist im Zusammenhang mit der erfindungsgemäßen Zusammensetzung beschrieben, daß diese topisch angewandt wird. Hierunter wird im vorliegenden Fall verstanden, daß die erfindungsgemäße Zusammensetzung sowohl auf der äußeren Haut als auch auf Schleimhäuten jeglicher Art zu applizieren ist.

Eine erste Weiterbildung der erfindungsgemäßen Zusammensetzung sieht vor, daß hierbei die innere Phase den Wirkstoff in einem Konzentrationsbereich zwischen 15 Gew.% und 85 Gew.%, vorzugsweise in einem Konzentrationsbereich zwischen 25 Gew.% und 75 Gew.%, und die Doppelmembranschicht den Wirkstoff in einem Konzentrationsbereich zwischen 85 Gew.% und 15 Gew.%, vorzugsweise in einem Konzentrationsbereich zwischen 75 Gew.% und 25 Gew.%, jeweils bezogen auf die Gesamtkonzentration an Wirkstoff, enthalten. Diese Weiterbildung der erfindungsgemäßen Zusammensetzung sieht im Vergleich zu der eingangs beschriebenen erfindungsgemäßen Zusammensetzung vor, daß hierbei gezielt der Wirkstoff auf beide Phasen, d.h. auf die innere Phase und die Doppelmembranschicht, in nennenswerten Konzentrationen verteilt wird, so daß dementsprechend beide Phasen dazu beitragen, daß die eingangs beschriebene Penetration des Wirkstoffes und damit auch die Permeation des Wirkstoffes, sofern dieser systemisch wirken soll, beeinflußt und optimiert werden.

Grundsätzlich besteht bei der erfindungsgemäßen Zusammensetzung die Möglichkeit, daß die Konzentration des Wirkstoffes in der inneren Phase und die Konzentration des Wirkstoffes in der der Doppelmembranschicht gleich sind. Besonders vorteilhaft ist es jedoch, wenn sich hier die Konzentrationen des Wirkstoffes in der inneren Phase und der Doppelmembranschicht unterscheiden, da hierdurch die Möglichkeit geschaffen wird, die Penetrationsgeschwindigkeit des Wirkstoffes zu beeinflussen.

Wie bereits zuvor bei der erfindungsgemäßen Zusammensetzung ausgeführt ist, weist die äußere Phase, die die spezielle Struktur, bestehend aus mindestens zwei Doppelmembranschichten mit einer dazwischen angeordneten Schicht der inneren Phase, umgibt, den Wirkstoff in einer Konzentration von bis zu maximal 5 Gew.%, bezogen auf die Gesamtkonzentration an Wirkstoff, auf. Besonders geeignet ist es jedoch, wenn die äußere Phase eine Wirkstoffkonzentration von bis zu maximal 3 Gew.% und insbesondere eine Wirkstoffkonzentration zwischen 2 Gew.% und 1 Gew.% sowie vorzugsweise eine Wirkstoffkonzentration zwischen 1 Gew.% und 0 Gew.%, jeweils bezogen auf die Gesamtkonzentration an Wirkstoff, enthält, da mit abnehmender Wirkstoffkonzentration in der äußeren Phase die Reproduzierbarkeit der Penetration des Wirkstoffes und damit auch dessen Permeation, sofern der Wirkstoff systemisch wirkt, gezielt beeinflußt werden kann. Desweiteren wird bei dieser Weiterbildung der erfindungsgemäßen Zusammensetzung sichergestellt, daß der Wirkstoffabbau, von dem insbesondere der in der äußeren Phase enthaltene Wirkstoff betroffen ist, erheblich reduziert wird, was wiederum einen Einfluß auf die Lagerstabilität der erfindungsgemäßen Zusammensetzung hat.

Eine andere Ausgestaltung der erfindungsgemäßen Zusammensetzung sieht vor, daß hierbei die Zusammensetzung einen hydrophoben Wirkstoff enthält, wobei der hydrophobe Wirkstoff überwiegend, vorzugsweise zu mindestens 70 Gew.% und insbesondere zu mindestens 80 Gew.%, bezogen auf die Gesamtkonzentration an Wirkstoff, innerhalb der Doppelmembranschicht angeordnet ist. Vorzugsweise sind solche Ausgestaltungen der erfindungsgemäßen Zusammensetzung möglich, bei denen der hydrophobe Wirkstoff in einer Konzentration zwischen 80 Gew.% und 90 Gew.%, bezogen auf die Gesamtkonzentration an Wirkstoff, innerhalb der Doppelmembranschicht eingelagert ist, wobei durch eine derartige Einlagerung in die Doppelmembranschicht, die Bestandteil der speziellen Struktur der erfindungsgemäßen Zusammensetzung ist, der Wirkstoff besonders gut gegen Alterungs- und/oder Umwelteinflüsse geschützt wird. Bei dieser speziellen Ausgestaltung, bei der der hydrophobe Wirkstoff in einer Konzentration zwischen 80 Gew.% und 90 Gew.%, bezogen auf die Gesamtkonzentration an Wirkstoff, der in der erfindungsgemäßen Zusammensetzung vorgesehen ist, eingelagert ist, weist somit die innere Phase eine maximale Konzentration an Wirkstoff zwischen 20 Gew.% und 10 Gew.%, bezogen auf die Gesamtkonzentration an Wirkstoff innerhalb der erfindungsgemäßen Zusammensetzung, auf, sofern die äußere Phase wirkstofffrei ist. Während der Anwendung einer derartigen Ausführungsform der erfindungsgemäßen Zusammensetzung findet dann der Wirkstofftransport aus der Doppelmembranschicht in die Oberflächenlipide und/oder Interzellular-Lipide statt, so daß gleichzeitig der Anteil an Wirkstoff, der aus der erfindungsgemäßen Zusammensetzung aus der Doppelmembranschicht abtransportiert wird, durch die innere Phase in die Doppelmembranschicht nachgeliefert wird. Dies führt über einen gewissen Zeitraum dazu, daß die Wirkstoffkonzentration innerhalb der Doppelmembranschicht konstant bleibt, mit der Folge, daß die Wirkstofftransportrate aus der Doppelmembranschicht in die zuvor genannten Hautlipide konstant bleibt.

Eine andere, besonders geeignete Ausführungsform der erfindungsgemäßen Zusammensetzung sieht vor, daß hierbei ein hydrophiler Wirkstoff enthalten ist, wobei der hydrophile Wirkstoff überwiegend, vorzugsweise zu mindestens 70 Gew.% und insbesondere in einem Konzentrationsbereich zwischen 80 Gew.% und 90 Gew.%, bezogen auf die Gesamtkonzentration an Wirkstoff, innerhalb der inneren Phase angeordnet ist. Auch diese Ausgestaltung erlaubt es, wie zuvor für den hydrophoben Wirkstoff beschrieben, innerhalb eines bestimmten Zeitraumes eine gleichbleibende Transportrate des Wirkstoffes zur Haut sicherzustellen.

Die zuvor beschriebene Wirkstoffverteilung zwischen der Doppelmembranschicht und der Schicht der inneren Phase richtet sich bei der erfindungsgemäßen Zusammensetzung zunächst danach, welcher Wirkstoff innerhalb der Struktur, die die erfindungsgemäß Zusammensetzung auszeichnet, eingelagert werden soll, wobei hierfür neben zwischenmolekularen Wechselwirkungen des Wirkstoffes mit dem Material der Doppelmembranschicht und dem Material der inneren Phase auch die Art des jeweiligen Wirkstoffes entscheidend ist. Handelt es sich z.B. um einen Wirkstoff, der in gleichem Maße lipophile und hydrophile Eigenschaften besitzt, so wird sich ein derartiger Wirkstoff vorzugsweise zu 50 Gew.% innerhalb der Doppelmembranschicht und zu 50 Gew.% innerhalb der inneren Phase, sofern diese innere Phase hydrophil ist, einlagern, wobei sich diese Konzentrationsangaben auf die Gesamtkonzentration des Wirkstoffes innerhalb der erfindungsgemäßen Zusammensetzung beziehen. Durch Variation der Lipophilie des Materials, das die Doppelmembranschicht ausbildet und durch Variation der Hydrophilie des Materials, das die innere Phase ausbildet, läßt sich dieses zuvor beschriebene Verteilungsgleichgewicht verschieben, so daß dementsprechend eine Erhöhung der Wirkstoffkonzentration in dem Material, das die Doppelmembranschicht ausbildet und eine Verringerung der Konzentration des Wirkstoffes in dem Material, das die Schicht der inneren Phase ausbildet, und naturgemäß umgekehrt, stattfindet.

Eine weitere Möglichkeit, die Wirkstoffverteilung zwischen der Doppelmembranschicht und der Schicht der inneren Phase bei der erfindungsgemäßen Zusammensetzung zu beeinflussen, sieht vor, daß hierbei der Wirkstoff innerhalb der Zusammensetzung mittels einer lipophilen Verbindung (Ankergruppe) an oder in der Doppelmembranschicht verankert bzw. eingelagert ist. Insbesondere durch Variation der stöchiometrischen Verhältnisse von lipophiler Verbindung zum Wirkstoff und durch Auswahl und Abstimmung der lipophilen Verbindung auf den jeweiligen Wirkstoff bzw. dem Material, aus dem die Doppelmembranschicht ausgebildet wird, kann die lipophile Verbindung in der Doppelmembranschicht eingelagert und somit fixiert werden und der hiermit verankerte Wirkstoff wahlweise an der Grenzschicht zwischen der Doppelmembranschicht und der Schicht der inneren Phase angeordnet werden, wobei besonders bevorzugt ist, wenn die lipophile Verbindung in der Doppelmembranschicht eingelagert und der hiermit verankerte Wirkstoff innerhalb der inneren Phase angeordnet ist.

Bei der zuvor beschriebenen Ausführungsform der erfindungsgemäßen Zusammensetzung, bei der der Wirkstoff mittels einer lipophilen Verbindung verankert wird, wird vorzugsweise eine solche lipophile Verbindung vorgesehen, bei der der Wirkstoff, der vorzugsweise ein hydrophiler Wirkstoff und/oder ein amphiphiler Wirkstoff ist, über zwischenmolekulare Wechselwirkungen, insbesondere über Wasserstoffbrückenbindung oder über Van-der-Waals-Kräfte, an der lipophilen Verbindung fixiert. Ein derartig fixierter Wirkstoff wird dann bei Anwendung dieser Ausführungsform der erfindungsgemäßen Zusammensetzung verzögert nur freigegeben, so daß diese Ausgestaltung der erfindungsgemäßen Zusammensetzung eine gute Depotwirkung besitzt.

Soll über eine derartige Verbindung (Ankergruppe) der hydrophile und/oder amphiphile Wirkstoff in der vorstehend beschriebenen Art an die Doppelmembranschicht fixiert werden, bieten sich hierfür solche Verbindungen an, die einerseits noch reaktive Gruppen aufweisen, über die der jeweilige Wirkstoff an der Verbindung (Ankergruppe) angekoppelt wird und die andererseits noch eine gewisse Lipophilie besitzen, um die zuvor beschriebene Ein- und/oder Anlagerung dieser Verbindung in und/oder an der Doppelmembranschicht bewirken. Geeignete Verbindungen sind somit insbesondere organische amphiphile Substanzen oder organische Substanzen mit entsprechenden reaktiven Zentren, so vorzugsweise alle längerkettigen Kohlenwasserstoffverbindungen, seien sie linear oder cyclisch (mono- und polycyclisch, homo- und heterocyclisch), die zusätzlich mit Halogen-, Hydroxy-, Säuren-, Ester-, Säureamid-, Amino-, Imino-, Säureimid- und/oder sonstigen polaren Gruppen, versehen sind. Als spezielle Verbindungen sind hier bevorzugt gesättigte und/oder ungesättigte C₃-C₂₄-Mono-bis Tripeptide, Alkanolamide, insbesondere Ethanolamine der C₁₄-C₂₄-Fettsäuren, C₁₀-C₂₄-Fettsäuren (gesättigt und ungesättigt), C₁₀-C₂₄-Fettsäuresalze, C₁₀-C₂₄-Fettalkohole und/oder C₁₀-C₂₄-Fettsäureester zu nennen.

Grundsätzlich besteht bei der erfindungsgemäßen Zusammensetzung die Möglichkeit, daß das Material der inneren Phase und das Material der äußeren Phase unterschiedlich ist, wobei jedoch bevorzugt, insbesondere unter dem Gesichtspunkt einer längeren Lagerstabilität der erfindungsgemäßen Zusammensetzung, daß die innere Phase und die äußere Phase vom Material her identisch sind. Insbesondere wird als Material für die äußere Phase und vorzugsweise somit auch als Material für die innere Phase jeweils eine Flüssigkeit ausgewählt, wobei der Begriff Flüssigkeit alle flüssigen Systeme abdeckt, deren Viskosität insbesondere zwischen niederviskos bis hochviskos variiert und somit nicht nur die Viskosität von eigentlichen Flüssigkeiten sondern auch die Viskosität von gelartigen Zubereitungen, insbesondere Oleogele, und auch Schäume umfaßt.

In Weiterbildung der zuvor beschriebenen Ausführungsform der erfindungsgemäßen Zusammensetzung, bei der die innere Phase und die äußere Phase jeweils eine Flüssigkeit sind, sieht eine Abwandlung dieser Ausführungsform vor, daß es sich bei dieser Flüssigkeit jeweils um Wasser handelt. Hierbei umfaßt dieser Begriff Wasser nicht nur destilliertes Wasser, entionisiertes Wasser oder osmotisch gereinigtes Wasser sondern deckt auch alle wäßrigen Systeme, so beispielsweise auch Puffersysteme oder Salzlösungen oder solche wäßrigen Systeme ab, die neben Wasser noch mit Wasser mischbare, physiologisch unbedenkliche organische Lösungsmittel enthalten.

Wie bereits zuvor wiederholt angesprochen, weist die erfindungsgemäßen Zusammensetzung einen lokal wirkenden Wirkstoff und/oder einen systemisch wirkenden Wirkstoff auf, wobei bei Auswahl eines lokal wirkenden Wirkstoffes die zuvor bei der erfindungsgemäßen Zusammensetzung wiedergegebenen Aussagen zum Penetrationsvermögen und bei einem systemisch wirkenden Wirkstoff die zuvor wiedergegebenen Aussagen bezüglich einer Penetration und Permeation des Wirkstoffs zu berücksichtigen sind.

Weist die erfindungsgemäßen Zusammensetzung einen pharmazeutischen Wirkstoff auf, so handelt es sich hierbei vorzugsweise um einen solchen pharmazeutischen Wirkstoff, der aus der Gruppe ausgewählt ist, die Analgetika, Antirheumatika, Antiallergika, Antibiotika, Antimykotika, Antiphlogistika, Balneotherapeutika, corticoide Wirkstoffe, Antiseptika, durchblutungsfördernde Wirkstoffe, Sedativa, Anästhetika, Spasmolytika, Wundbehandlungsmittel, Antipruriginosa, wie insbesondere Polidocanol, Benzocain und/oder Lidocain, Antipsoriatica, wie insbesondere Sphingosin-1-phosphat, Dithranol und/oder Becocalcidiol, Anti Acne Mittel, wie insbesondere Benzoylperoxid, Doxicyclin und/oder Vitamin A-Säure, Anti Rosazea Mittel, wie insbesondere Metronidazol und/oder Vitamin K, Antiherpetica, wie insbesondere Acyclovir, Haemorrhoidenmittel, wie insbesondere Bufexamac und/oder Lidocain, Venentherapeutica, wie insbesondere Heparinoide und/oder Roßkastanienextrakt, Immunmodulatoren, wie insbesondere Tacrolimus und/oder Pimecrolimus, Mittel zur Behandlung von Hautkrebs, wie insbesondere 5-Fluorouracil und/oder Cyclooxygenase-2 Inhibitoren, jeweils allein oder in Mischung, umfaßt. Hierbei wird der jeweilige pharmazeutische Wirkstoff danach ausgewählt, welche therapeutische Aufgabe die erfindungsgemäße Zusammensetzung bei ihrer topischen Anwendung zu lösen hat, wobei die zuvor aufgeführten bevorzugten Wirkstoffe, sofern sie miteinander verträglich sind, auch als Mischung appliziert werden können. Der besondere Vorteil einer derartigen Ausgestaltung der erfindungsgemäßen Zusammensetzung, die einen pharmazeutischen Wirkstoff enthält, liegt darin, daß mit einer topischen Applikation der erfindungsgemäßen Zusammensetzung selbst dann Hautreizungen und Hautirritationen vermieden werden, wenn der jeweils in der Zusammensetzung vorhandene Wirkstoff dafür bekannt ist, daß er entsprechende Hautreizungen bzw. Hautirritationen hervorruft.

Geeignete Analgetika, die insbesondere systemisch wirken, werden insbesondere aus der Gruppe der nicht-opioiden Analgetika ausgewählt und umfassen vorzugsweise die an sich bekannte Salicylsäurederivate, wie insbesondere Acetylsalicylsäure, Amide der Salicylsäure, Salsalate, Benorilate und Difunisale, Anilin-Derivate, wie insbesondere Paracetamol, Phenacetin, Anthranilsäure-Derivate, wie insbesondere Mefenaminsäure, Flufenaminsäure, Nilfuminsäure, Pyrazol-Derivate, Azapropazone sowie Heteroaryl- und Aryl-essigsäuren und Arylpropionsäuren.

Bei den Antimykotika sind insbesondere als pharmazeutische wirkstoffe die topisch anzuwendenden Azolderivate, wie insbesondere Fenticonazol, Clotrimazol, Econazol, Isoconazol, Ketoconazol, Miconazol, Oxiconazol, Tioconazol, Flutrimazol, die Polyene, wie insbesondere Nystatin, die Cicloprioxolamin, wie insbesondere Ciclopirox, die Allylamine, wie insbesondere Naftifin, Terbinafin, und/oder die Morpholine, wie insbesondere Amorolfin, zu nennen.

Bei den corticoiden Wirkstoffe sind insbesondere Cortison, Hydrocortison, Glucocorticoide und deren Derivate wie Triamcinolonacetonid sowie andere Cortison-Derivate, die insbesondere bei der topischen Applikation eine lokale Wirksamkeit besitzen, zu nennen.

Desweiteren kann, je nach Anwendungsgebiet der erfindungsgemäßen Zusammensetzung, als pharmazeutischer Wirkstoff ein antiinflammatorischer Wirkstoff, so insbesondere, Bufexamac, Kamillenextrakt, Hamamelisextrakt, Gerbstoffe, Bisabolol, Ammoniumbituminosulfonat oder Allantoin, ein Immunsupresiva, wie insbesondere Methotrexat, Ciclosporin, Reinoide, vorzugsweise Isotretinoin, Acitretinoin oder Tazaroten, oder Antiinfektiva, wie insbesondere Clindamycin, Tetracycline, oder ein Antiseptika, wie insbesondere Chlorhexidin, Benzalkoniumchlorid, 8-Hydroxychinoline, Ethacridin, Hexatidin, Acriflaviniumchlorid, Benzoxoniumchlorid, Bibrocathol, Dequaliniumsalze, Azelainsäure, Resorcin, Triclosan, Farnesol, Diglycerinmonocaprinat, kolloidales Silber, Silbersalze, wie insbesondere Silbercitrat, Silbernitrat und/oder Silberchlorid, oder Gentamycin, oder Virustatika enthalten sein, wobei selbstverständlich die erfindungsgemäße Zusammensetzung auch mehrere der zuvor aufgeführten Wirkstoffe aufweisen kann.

Zusätzlich zu den zuvor aufgeführten pharmazeutischen Wirkstoffen oder anstelle der zuvor aufgeführten pharmazeutischen Wirkstoffen sieht eine besonders geeignete Ausführungsform der erfindungsgemäßen Zusammensetzung vor, daß hierbei die erfindungsgemäße Zusammensetzung mindestens einen kosmetischen Wirkstoff enthält, der aus der Gruppe ausgewählt ist, die Öle, Fette, Wachse, Antioxidantien, Peptide, Proteine, Aminosäuren, Derivate der Aminosäuren, Lichtschutzfilter, Bräunungsmittel, Vitamine, Pro-Vitamine, Fruchtsäuren, Feuchthaltesubstanzen, Pflanzenteile und Pflanzenextrakte, Harnstoff, Glucane, Glucanderivate, organische Metallverbindungen und anorganische Metallverbindungen umfaßt.

Lichtschutzfilter, die auch bisweilen als Sonnenschutzfilter insbesondere in kosmetischen Zusammensetzungen bezeichnet werden, werden bevorzugt aus der Gruppe ausgewählt, die PABA und Derivate (= PEG-25, PABA), Octyl dimethyl PABA, Homosalate, Oxybenzon BEMT, p-Methoxycinnamat, Ethylhexyl Triazone, Octocrylen, Benzophenon-3, Benzophenon-4, Benzophenon-9, Diethylamino-hydroxybenzoylhexylbenzoat, Drometrizole Trisiloxan, 4-Methylbenzylidene Campher, 3-Benzylidene Campher, Octylsalicylat, Methylene Bis-Benzotriazolyl Tetramethylbutylphenol und Bis-Ethylhexyloxyphenol Methoxyphenyl Triazin, Ethylhexylmethoxycinnamate, Diethylhexyl Butamido Triazone, Phenylbenzimidazol Sulfonic Acid, Butyl Methoxydibenzoylmethane, Diethylamino Hydroxybenzoyl Hexyl Benzoate, Disodium Phenyl Dibenzimidazole Tetrasulfonate und Terephthalylidene Dicamphor Sulfonic Acid, umfaßt.

Als Antioxidantien sind insbesondere als Einzelsubstanz oder als Mischung in der erfindungsgemäßen Zusammensetzung Vitamine, insbesondere Vitamin A und/oder Vitamin C, Tocopherole, Carcinin, Liponsäure, Liposol Maleate, Carotenoide, Lycopene, farblose Carotenoide, insbesondere das aus der Tomate isoliere IBR -TCLC oder das aus Algen isolierte IBR-CLC, Polyphenole, wie vorzugsweise Epicatechine, Epigallocatechine, Epigallocatechingallat und/oder Epicatechnin-3-gallate, Kaffeesäure, Kaffeesäureester, Romarinsäure, Flavonoide, die vorzugsweise aus Tee, Wein, Kaffee, Kakao, Rooisbos, Cocoa oder Weintraubenkernextrakt isoliert werden, so insbesondere Flavanole, Flavanone, Anthocyanidine, Proanthocyanidine, Resveratrol, Silymarin, Aspalathin, Ellaginsäure, Curcuminderivate, Dihydroquercetin, N.D.G.A., Rutin, Tetrahydrocurcuminoid, Tetrahydrodiferuloylmethane, Tetrahydrodemethoxydiferuloylmethane, Tetrahydrobisdemethoxydiferuloylmethane, Glutathione, Coenzyme q 10, L-Carnosin, N-Acetylcystein, Phytinsäure, Furalglucytol, Chelatbildner, so insbesondere Thioctinsäure und/oder EDTA, BHA, BHT, SOD, 4-Thiazolidinonekinetin. Darüber hinaus kann die erfindungsgemäße Zusammensetzung noch pflanzliche Inhaltsstoffe aufweisen, die insbesondere durch Extraktion von Pflanzen, von Pflanzenteilen, von Früchten, von Schalen und/oder von Samen von Rosmarin, Hopfen, Ingwer, Picea abies extract und daraus isolierte Lignan-Leitsubstanzen, wie insbsondere Hydroximatairesinol, Matairesinol und Seccoisolaricirinol, Picea Abies Extrakt, Pinus pinaster, Pycnogenol, Uniprotect PT-3, Unirepair T-43 (Hersteller: Induchem), Bakuchiol, Coffea arabica, Quercus infectoria, Camelia sinensis, Olea europea, Rosmarinus officinlis, Artemisia Umbellifloris, Buddleja Davidii, Leontopodium Alpinum oder durch Extraktion von Algen hergestellt werden.

Die bevorzugten Peptide werden vorzugsweise aus der Gruppe ausgewählt, die Pentapeptide, Hexapeptide, insbesondere Hexapeptide-2 und/oder Hexapeptide-9, Heptapeptide, Kupfer-Peptide, Wachstumsfaktoren der TGF Beta Familie, MPC Milch Peptide, MTP Milch Tripeptide, Palmitoyloligopeptide/Matrikine, insbesondere Pal-KTTKS (Hersteller: Sederma) und/oder Pal-VGVAPG (Hersteller: Sederma), Acetylhexapeptide 3, Palmitoylpentapeptide, Palmitoyltripeptide-5, Serilesine (= Laminin, Hersteller: Lipotec), Lipeptide (= Oligopeptide, Hersteller: Lipotec), Tripeptide 10-citrullin, Aldenine (Hersteller: Lipotec), Myoxinol (Hersteller: Cognis), Tripeptide-1, Tripeptide-3, Hexapeptide-9, Hexapeptide-2, Oligopeptide-6, Dipeptide-4, Decapeptide-2, Phytoquintescine (Hersteller: Vincience), Glutathion, Cytokin, Soja oligopeptide, Polygamma-Glutamine-Säure, enthalten.

Bevorzugte Proteine werden aus der Gruppe ausgewählt, die Collagen, Collagenderivate, Antarcticin (= Glycoprotein, Hersteller: Lipotec), Keratin, hydrolisiertes Weizenprotein, Sojaprotein, vorzugsweise hydrolisiertes und/oder extrahiertes Sojaprotein, Elastin und Reiskeimprotein umfaßt.

Besonders geeignete Aminosäuren bzw. deren Derivate sind Lysin, Alanin, Serin, Glycin, Arginin, Glutaminsäure, Histidin, Valin, Cystein und/oder Aminoguadin, wobei diese Aminosäure bzw. die entsprechenden Derivate insbesondere auch als Feuchthaltsubstanzen in der erfindungsgemäßen Zusammensetzung enthalten sind. Weitere Feuchthaltesubstanzen umfassen vorzugsweise Caprylyl Glycol, Urocaninsäure, Creatin, Glucosamin, Hyaluronsäure, Hyaluronsäure Ectoin, Trehalose, Lactobionsäure, Taurin, Xylitylglucoside anhydroxylitol xylitol (Hersteller: Seppic), Wasserporin 3-synthese-stimulatoren, wie insbesondere Opuntia Extrakt, Milchsäure, Pyrrolidoncarbonsäure, Alpha-Hydroxysäuren oder Beta-Hydroxysäuren, wie insbesondere Hydroxycarbonsäuren, Dicarbonsäuren, insbesondere Gluconsäure, Citronensäure, Apfelsäure, Weinsäure, deren Derivate und/oder deren Salze.

Bei den Ölen, die unter anderen als kosmetischer Wirkstoff in der erfindungsgemäßen Zusammensetzung enthalten sind, sind insbesondere Wiesenschaumkrautöl, Avocadoöl, Kokosnußöl, Jojobaöl, Weizenkeimöl, Macadamianußöl, Aprikosenkernöl, Hanföl, Leinsamenöl, Sesamöl, Sonnenblumenöl, Erdnußöl, Rosmarienöl, Kamillenöl, Salbeiöl, Calendulaöl, Lavendelöl, Johanniskrautöl, Melissenöl, Sanddornöl, Teebaumöl, Zedernholzöl, Zypressenöl, Nachtkerzenöl, Johannisbeerkernöl, Borretschöl, Hagebuttenöl, Sojaöl, Fischöl, Mandelöl, Olivenöl, Palmöl, Distelöl, Moringa Samenöl, Rizinusöl, Süssmandelöl, Maiskeimöl, Canolaöl, Arganöl, Amaranth Samenöl, und/oder Bestandteile dieser Öle zu nennen. Unter den Begriff Bestandteile dieser Öle fallen insbesondere solche Ölfraktionen, die sich durch einen einheitlichen und standardisierten Aufbau, durch ihren Grad der Sättigung und/oder durch die Anzahl der Doppelbindung spezifiziert sind.

Die Konzentration an Öl bzw. Ölbestandteil, das bzw. der als kosmetischer Wirkstoff in der erfindungsgemäßen Zusammensetzung für deren kosmetische Anwendung enthalten ist, richtet sich nach dem jeweiligen Anwendungsgebiet und variiert insbesondere zwischen 0,5 Gew.% und 40 Gew.%, bezogen auf die anwendungsfertige Zusammensetzung.

Zu den zuvor aufgeführten organischen und anorganischen Metallverbindungen, die als kosmetische Wirkstoffe in Ausführungsformen der erfindungsgemäßen Zusammensetzung enthalten sind, gehören insbesondere Natrium-, Kalium-, Magnesium-, Calcium- und Zinksalz, wobei hier vorzugsweise als Anionenfluoride, Fluoride, Sulfate, Phosphate, 2-Aminoethylphosphate, Glycolate, Lactate, Fumarate, insbesondere Monomethyl- und/oder Monoethylfumarate, Tartrate, jeweils allein oder in Mischung. Desweiteren kann, je nach Anwendungsgebiet, die erfindungsgemäße Zusammensetzung natürliche Meersalze als kosmetischen Wirkstoff enthalten. Darüber hinaus kann in der erfindungsgemäßen Zusammensetzung als anorganische bzw. organische Verbindung Magnesiumoxid, Magnesiumcarbonat, Magnesiumaluminiumsilikat, Magnesiumstearat, Magnesiumisostearat, Talkum, Calciumcarbonat, Zinkoxid, Zinkcarbonat, Zinkstearat, Zinklaurat, Titandioxid, Eisenoxid, Eisenhexacyanoferrat, Wismutoxyclorid, Aluminiumoxid, Alumosilikate oder Siliciumdioxid vorhanden sein, wobei als Bräunungsmittel die kosmetisch zugelassenen Farbstoffe und/oder bräunungsbeschleunigende Mittel, wie insbesondere Dihydroxyaceton und/oder Erythrulose zu nennen ist.

An Stelle der zuvor genannten Feuchthaltesubstanzen oder zusätzlich hierzu enthalten andere Ausgestaltungen der erfindungsgemäßen Zusammensetzung als kosmetische Wirkstoffe solche Feuchthaltesubstanzen, die insbesondere physiologisch verträgliche Polyole, wie vorzugsweise Glykol, Propylenglykol, Butylenglykol, Pentylenglykol, Hexylenglykol und/oder Glycerin, Saccharide, wie insbesondere Inositol, Sorbitol, Mannit, Platinit, Maltrodextrin, Dextrin, Cylclodextrin, Glucose, Fructose, Lactose, Mannose, Glaktose, Decylen Glykol und/oder Octandiol umfassen. Eine besonders bevorzugte Feuchthaltesubstanz, die in der erfindungsgemäßen Zusammensetzung als kosmetischer Wirkstoff enthalten ist, sind Harnstoffe sowie Harnstoffderivate.

Neben den bereits zuvor aufgeführten Öle bzw. Ölbestandteile sind desweiteren Fette und Wachse als kosmetische Wirkstoffe zu nennen, so insbesondere Reiskeimwachs, Mono-, Di-, Tri-und/oder Polyglyceride der Rizinolsäure, der 12-Hydroxystearinsäure und/oder der 11-Hydroxypalmitinsäure, Rizinolsäureoctyldedecylester, 12-Hydroxystearinsäureoctylester, Bienenwachs, Japanwachs, Carnaubawachs, Cetylpalmitat, Kakaobutter, Sheabutter, Squalan, Cholesterin, Cholesterylsulfat, Phytosterole und/oder Lanolin, insbesondere Lanolinalkohole oder Derivate. Bevorzugt sind auch Wachse und Öle der Pflanzengattung Peciloneuron Indicum und insbesondere solche, die mindestens 20 Gew.% Lignocerinsäure enthalten, sowie ferner synthetische und natürliche Mischungen epidermaler Lipide, wie diese unter der Bezeichnung Skinmimimics von der Firma Degussa und Meadowestolide von der Firma Fancor angeboten werden. Desweiteren kann, je nach Anwendungsgebiet, als kosmetischer Wirkstoff in der erfindungsgemäßen Zusammensetzung Vitamine und/oder Pro-Vitamine, insbesondere Vitamin A, Vitamin B-Komplex, Vitamin C, Vitamin E sowie Vitamin D und/oder deren Derivate, wie insbesondere Vitamin A-Säure, Vitamin A-Acetat, Vitamin A-Palmitat, Vitamin C-Palmitat, Vitamin E-Acetat, Vitamin E-Palmitat und/oder Vitamin E-Linoleat, Alfacalcidol, Calcitriol, Colecalciferol, Ergocalciferol, Transcalcifediol, Calciprotriol, Calcifediol, Vitamin D₃, β-Carotin, Panthenol, Pantothensäure, Biotin oder aber auch juckreizstillende Oberflächenanästhetika, so insbesondere Lidocain, Benzocain, Polidocanol, wäßrige Harnstofflösungen, Isoprenalin, Cortamiton, Quinisocain, juckreizstillende H₁-Antihistaminika, so insbesondere Meclozin, Cetirizin, Promethazin, Diphenhydramin, Clophenoxamin, Doxylamin, Pheniramin, Dexchlorpheniramin, Bamipin, Clemastin, Dimetiden, Mebhydrolin, Loratadin, Oxatomid, Terfenadin und/oder Astemizol enthalten sein. Bei den Glucanderivaten ist insbesondere das Carboxymethylglucan oder Carboxymethylglucan zu nennen.

Eine besonders geeignete Weiterbildung der erfindungsgemäßen Zusammensetzung, die insbesondere einen pflegenden kosmetischen Wirkstoff ausweist, sieht vor, daß dieser Wirkstoff, der selbstverständlich auch ein Wirkstoffgemisch sein kann, aus der Gruppe ausgewählt ist, die Sheabutter, Ceramide, insbesondere Ceramide-1, Ceramide-3, Ceramide-6 und/oder Ceramide-7, Cupuacu-Butter, Squalan und/oder Triglyceride, insbesondere mittelkettige, gesättigte C₈-C₂₄-Triglyceride, umfaßt. Diese Weiterbildung der erfindungsgemäßen Zusammensetzung ist insbesondere dazu geeignet, die Interzellular-Lipide der Haut zu stärken, aus- und aufzubauen sowie den Säuremantel und die Menge des Hautoberflächenfettes zu vergrößern und damit einen erhöhten Schutz der Haut zu bewirken.

Um mit der erfindungsgemäßen Zusammensetzung insbesondere infizierte, gereizte oder erkrankte Hautbereiche, so zum Beispiel Ekzeme, Brandwunden, vorzugsweise 1. und 2. Grades, Dekubitus, oder Abszesse zu behandeln oder wirksam die Haut vor derartigen Erkrankungen zu schützen, sieht eine Weiterbildung der erfindungsgemäßen Zusammensetzung vor, daß hierin alternativ oder zusätzlich zu den zuvor genannten Wirkstoffen, insbesondere den zuvor genannten kosmetischen Wirkstoffen mindestens ein anti-entzündlicher Wirkstoff enthalten ist, der ausgewählt ist aus der Gruppe, die Ursolsäure, Soja-Sterol, 18-beta-Glycyrrhetinsäure, Gamma-Oryzanol, Ferula-Säure, Avenanthramide und Derivate der zuvor genannten anti-entzündlichen Wirkstoffe umfaßt.

Insbesondere ist in einer derartigen Ausführungsform der erfindungsgemäßen Zusammensetzung der Wirkstoff in einer Kornzentration zwischen 0,001 Gew.% und 35 Gew.%, vorzugsweise in einer Konzentration zwischen 0,1 Gew.% und 15 Gew.%, bezogen auf die anwendungsfertige Zusammensetzung, vorhanden, wobei diese sich Konzentrationsangaben vorzugsweise auf die zuvor genannten und nachfolgend noch aufgeführten kosmetischen Wirkstoffe beziehen. Solche Ausgestaltungen der erfindungsgemäßen Zusammensetzung, die pharmazeutisch angewandt werden und die eingangs genannten pharmazeutischen Wirkstoffe und den zuvor aufgeführten anti-entzündlichen Wirkstoff enthalten, weisen Wirkstoffkonzentrationen auf, die insbesondere zwischen 0,01 Gew.% und 5 Gew.% und vorzugsweise zwischen 0,1 Gew.% und 2,5 Gew.%, bezogen auf die anwendungsfertige Zusammensetzung, variieren.

Alle die zuvor als kosmetische Wirkstoffe aufgeführten pflanzlichen Öle oder pflanzlichen Extrakte können auch durch entsprechende pflanzliche Teile, so insbesondere Wurzeln, Samen oder Blüten, ersetzt werden, sofern diese pflanzlichen Teile entsprechend getrocknet und zerkleinert, insbesondere pulverisiert, werden.

Zu den bevorzugten weiteren kosmetischen Wirkstoffen, von denen mindestens einer in der der erfindungsgemäßen Zusammensetzung enthalten sein muß, gehören die "Anti-Age"-Wirkstoffe auf der Basis der zuvor genannten Peptide und Proteine, Metallproteinaseinhibitoren, Seneszenzverzögerer, so insbesondere Geranylgeranon, und Niacinamid, hautregenerationsfördernde Wirkstoffe, wie insbesondere Retinol, Retinolderivate, Hefeextrakte, Panthenol, Allantoin, DNA Repairfördernde Substanzen, wie insbesondere T 4 Endonuklease V Enzyme, andere Enzyme, wie z.B. Zonase (Hersteller: Wasser Bio Technologie), weitere barriereunterstützende Wirkstoffe, wie vorzugsweise Calciumverbindungen insbesondere Calciumpanthothenat, Hydroxiapatit und ihre Mischungen, SodiumBeta Sitosterol Sulfate, Glycyrrhetinsäureverbindungen, Bisabolol, Antiirritantien, wie Antifreeze Proteine, so z.B. AAGP™ (Hersteller: Protokinetix), Chitosan, Hautbleichmittel, wie z.B. Arbutin, Anticellulite Wirkstoffe, insbesondere Koffein, Wirkstoffe zur Narbenbehandlung, wie z.B. Panthenol, Harnstoff, Heparin und/oder spezielle Pflanzenextrakte, Deodorantien/Antitranspirantien, so z.B. Aluminiumchlorohydrat, Aluminium-Zirkonium Chlorohydrat, Parfums, Mundpflegemittel, wie z.B. Chlorhexidinglukonat, Haarbehandlung, insbesondere Finasterid, Aminexil, Ketoconazol, Fußpflegemittel, wie z.B. Harnstoff und/oder Salicylsäure, Handpflegemittel und/oder Babypflegemittel, wie z.B. Allantoin und/oder Panthenol, Mittel zur Reduzierung oder Wachstumsverlangsamung unerwünschter Körperbehaarung, wie z.B. Eflornithin, Rasierhilfen, sowie Wirkstoffe zur Behandlung unreiner fettiger Haut und der damit verbundenen Begleiterscheinungen, antibakteriell wirkende und/oder sebumhemmende Wirkstoffe, wie insbesondere Salicylsäure und/oder Acnacidol (Hersteller: Vincience).

Eine weitere, besonders geeignete Ausführungsform der erfindungsgemäßen Zusammensetzung sieht vor, daß diese hierbei als mindestens einen Wirkstoff einen solchen Wirkstoff aufweist, der aus der Gruppe, bestehend aus Icaridin, Nelkenöl, Citronellal, Zedernholzöl, Lavelöl, Zimtöl, Permethrin und Crotamiton ausgewählt ist. Hierbei dienen diese Ausführungsformen der erfindungsgemäßen Zusammensetzung schwerpunktmäßig zur Prophylaxe von Insektenstichen, insbesondere Stichen von Mücken, Flöhen, Läusen und/oder Zecken.

Grundsätzlich ist festzuhalten, daß die erfindungsgemäße Zusammensetzung als Trägersubstanz, die mit der äußeren Phase und insbesondere mit Wasser die zuvor beschriebene spezielle Struktur ausbildet, solche Trägersubstanzen enthält, die gleichzeitig einen hydrophilen sowie einen hydrophoben Molekülrest aufweisen. Hierbei sind insbesondere bevorzugt solche Trägersubstanzen zu nennen, die aus der Gruppe ausgewählt sind, die Monoglyceride, Diglyceride, Triglyceride, bevorzugt auch destillierte mittelkettige Monoglyceride, Sphingolipide, Phosphatidylcholin, Phospholipide, Fettalkohole, Fettsäuren und Derivate der zuvor genannten Verbindungen umfaßt, wobei die Fettsäuren und Fettalkohole bevorzugt eine C₈-C₂₄-gesättigte lineare Kohlenstoffkette besitzen.

Besonders geeignet ist es jedoch, wenn in der erfindungsgemäßen Zusammensetzung als Trägersubstanz, die in der Lage ist, die zuvor mehrfach beschriebene spezielle Struktur auszubilden, mindestens ein hydriertes Lecithin und/oder ein hydriertes Phospholipid, und insbesondere ein hydriertes Phosphatidylcholin, enthalten ist. Hier konnte nämlich festgestellt werden, daß derartige hydrierte Phospholipide und insbesondere das hydrierte Phosphatidylcholin einerseits im hohen Maße mit der äußeren Phase diese speziellen Strukturen, die der erfindungsgemäßen Zusammensetzung eigen sind und diese charakterisieren, ausbildet und die andererseits hervorragend geeignet sind, in die interzellularen Lipide der Haut und insbesondere in die interzellularen Lipide der Hornschicht zu wandern und dort den Aufbau bzw. Ausbau dieser interzellularen Lipidschicht zu unterstützen, wie dies vorstehend wiederholt beschrieben ist. Ferner haben die hydrierten Lecithine und insbesondere das hydrierte Phosphatidylcholin den weiteren Vorteil, daß sie besonders stabile Zusammensetzungen ausbilden, die einerseits gegenüber chemischen und insbesondere oxidativen Angriff widerstandsfähig sind und andererseits eine hohe physikalische Stabilität und somit eine extrem große Lagerstabilität besitzen. Innerhalb dieser Struktur eingelagerte Wirkstoffe werden dementsprechend sehr wirksam gegen einen Abbau geschützt.

Bevorzugt wird jedoch ein solches hydriertes Lecithin bzw. ein solches hydriertes Phospholipid und insbesondere ein solches hydriertes Phosphatidylcholin in der erfindungsgemäßen Zusammensetzung vorgesehen, bei dem alle Acylreste ausschließlich oder überwiegend gesättigt sind, so daß insbesondere nur noch ungesättigte Acylreste in einer Konzentration kleiner als 10 Gew.% und vorzugsweise weniger als 5 Gew.% und ganz bevorzugt weniger als 1,5 Gew.% in dem hydrierten Lecithin, dem hydrierten Phospholipid und/oder insbesondere in dem hydrierten Phosphatidylcholin vorhanden sind.

Klarstellend sei angemerkt, daß der Begriff Phospholipid selbstverständlich nicht nur ein einzelnes Phospholipid sondern auch ein Gemisch von Phospholipiden abdeckt, wobei das Phospholipid bzw. das Phospholipidgemisch natürlichen oder synthetischen Ursprungs sein kann. Ebenso selbstverständlich ist es, daß das Phospholipid nicht nur im vorstehenden Sinne hydriert sein kann sondern daß anstelle dieses hydrierten Phospholipids ein synthetisches Phospholipid eingesetzt wird, bei dem die Acylreste im vorstehenden Sinne alle oder überwiegend gesättigt sind.

Die vorstehend beschriebenen Vorteile besitzen solche Weiterbildungen der erfindungsgemäßen Zusammensetzung im verstärkten Maße, die als Trägersubstanz ein hydriertes Phospholipid enthalten, das mindestens 60 Gew.% und vorzugsweise zwischen 70 Gew.% und 95 Gew.% hydriertes Phosphatidylcholin aufweist, wobei sich diese Konzentrationsangaben auf die Konzentration des hydrierten Phospholipids beziehen, das als solches in der anwendungsfertigen erfindungsgemäßen Zusammensetzung als Trägersubstanz enthalten ist.

Bezüglich der Konzentration der in der erfindungsgemäßen Zusammensetzung enthaltenen Trägersubstanz, die die der erfindungsgemäßen Zusammensetzung eigenen Struktur ausbildet, ist allgemein festzuhalten, daß sich diese Konzentration danach richtet, für welchen Zweck die erfindungsgemäßen Zusammensetzung angewendet wird und welchen Wirkstoff sie enthält. Desweiteren richtet sich die Konzentration der Trägersubstanz nach der chemischen Art der jeweils ausgewählten Trägersubstanz und ferner danach, welche Konzentration an Doppelmembranschichten innerhalb der erfindungsgemäßen Zusammensetzung enthalten sein soll. Insbesondere ist die mindestens eine Trägersubstanz in der erfindungsgemäßen Zusammensetzung in einer Konzentration zwischen 0,5 Gew.% und 30 Gew.%, vorzugsweise in einer Konzentration zwischen 0,7 Gew.% und 5 Gew.%, bezogen auf die anwendungsfertige Zusammensetzung, vorhanden.

Insbesondere dann, wenn das zuvor beschriebene hydrierte Phospholipid, das hydrierte Phosphatidylcholin bzw. das hydrierte Lecithin oder ein entsprechend synthetisch hergestelltes Phospholipid mit entsprechenden gesättigten Acylresten eine Phasenübergangstemperatur über 30 °C und unter 70 °C besitzt, läßt sich unter Verwendung einer derartigen Trägersubstanz besonders einfach solche Ausführungsformen der erfindungsgemäßen Zusammensetzung erstellen, die die erwünschte und eingangs umfangreich beschriebene spezielle Struktur aufweisen. Hierbei ist die Phasenübergangstemperatur so definiert, daß sie die Temperatur bezeichnet, an der das kristalline System der Trägersubstanz in ein flüssiges System der Trägersubstanz übergeht, wobei vielfach diese Temperatur keine konkrete Einzeltemperatur darstellt sondern durch einen Temperaturbereich gekennzeichnet ist. So liegt beispielsweise die Phasenübergangstemperatur für das besonders bevorzugte hydrierte Phosphatidylcholin, das aus Sojabohnen isoliert ist und das eine Phosphatidylcholin-Konzentration von 93 ± 3 Gew.% aufweist und dessen Acylreste zu 85 Gew.% aus Stearinsäure und zu 14 Gew.% aus Palmitinsäure bestehen, zwischen 54 °C und 58 °C und beträgt insbesondere 56 °C.

Wie bereits zuvor beschrieben ist, sieht eine besondere bevorzugte Ausführungsform der erfindungsgemäßen Zusammensetzung vor, daß diese als innere und äußere Phase Wasser aufweist. Hierbei variiert, je nach Anwendung, die Konzentration des Wasser in der erfindungsgemäßen Zusammensetzung zwischen 5 Gew.% und 90 Gew.%, bezogen auf das Gewicht der anwendungsfertigen Zusammensetzung, wobei diese Konzentrationen auch bevorzugt für andere Flüssigkeiten gelten, die die innere und/oder äußere Phase bilden.

Abhängig von dem jeweils vorgesehenen Anwendungsgebiet und der jeweils ausgesuchten Trägersubstanz sowie des jeweils anzuwendenden Wirkstoffs sieht eine vorteilhafte Weiterbildung der erfindungsgemäßen Zusammensetzung vor, daß diese mindestens einen Alkohol, insbesondere einen mehrwertigen Alkohol, aufweist, wobei selbstverständlich hier als Alkohole solche Alkohole ausgewählt werden, die keine oder nur eine äußerst geringe Hautirritation hervorrufen.

Als besonders geeignete Alkohole haben sich in der erfindungsgemäßen Zusammensetzung Pentylenglykol, Caprylyl Glykol, Phenylethylalkohol, Decylen Glykol, Glycerin oder Mischungen der zuvor genannten Alkohole erwiesen, so daß dementsprechend diese Alkohole und insbesondere die zuvor beschriebene Dreiermischung aus Pentylenglykol, Caprylyl Glykol und Glycerin in der erfindungsgemäßen Zusammensetzung enthalten sind.

Eine weitere vorteilhafte Ausgestaltung der erfindungsgemäßen Zusammensetzung sieht vor, daß diese zusätzlich zu den zuvor genannten Wirkstoffen oder alternativ hierzu noch mindestens ein N-Acyl-Alkanolamin und vorzugsweise N-Acyl-Ethanolamin enthält, wobei dieses N-Acyl-Alkanolamin dafür bekannt ist, daß es anti-entzündliche Eigenschaften besitzt. Hierbei variiert die Konzentration des N-Acyl-Alkanolamins und insbesondere des N-Acyl-Ethanolamins zwischen 0,01 Gew.% und 10 Gew.%, vorzugsweise zwischen 0,1 % und 3 %, jeweils bezogen auf das Gewicht der anwendungsfertigen Zusammensetzung.

Insbesondere dann, wenn das N-Acyl-Alkanolamin einen C₁-C₂₄-Acylrest, vorzugsweise einen linear gesättigten und/oder ungesättigten C₁-C₂₄-Acylrest, aufweist, lassen sich mit einer derartigen Weiterbildung der erfindungsgemäßen Zusammensetzung besonders gut extrem auftretende entzündliche Hautreizungen oder Hauterkrankungen behandeln. wobei insbesondere auch Hautreizungen, Hautirritationen, Hautrötungen und Hautbrennen bereits nach wenigen Anwendungen beseitigt werden.

Insbesondere wird in den zuvor beschriebenen Ausführungsformen der erfindungsgemäßen Zusammensetzung, die das N-Acyl-Alkanolamin enthalten, dieses N-Acyl-Alkanolamin aus der Gruppe ausgewählt, die N-Acetyl-Ethanolamin, N-Oleoyl-Ethanolamin, N-Linolenoyl-Ethanolamin, N-Cocoyl-Ethanolamin und N-Palmitoyl-Ethanolamin umfaßt, wobei diese zuvor genannten speziellen Ethanolamine sowohl als Einzelsubstanz als auch als Gemisch von mehreren Ethanolaminen Anwendung finden. Ebenso kann die erfindungsgemäße Zusammensetzung als N-Acyl-Alkanolamin ein N-Acyl-2-Hydroxy-Propylamin umfassen, wobei dieses N-Acyl-2-Hydroxy-Propylamin insbesondere als Acylrest Fettsäuren aus Kokosfett und/oder Palmöl enthält. Die zuvor aufgeführten N-Acyl-Alkanolamine bewirken desweiteren, daß die Hautfeuchtigkeit erhöht und zusätzlich auf einen akzeptablen Wert stabilisiert wird.

Abhängig von der jeweiligen Art der Applikation, d.h. ob die erfindungsgemäße Zusammensetzung z.B. als Creme, Salbe, Gel, Lotion oder Zusatz zum Bad, verwendet wird, enthalten entsprechend formulierte Ausführungsformen der erfindungsgemäßen Zusammensetzung mindestens ein Konservierungsmittel, ein Antioxidanz, ein Verdickungsmittel und/oder einen Gelbildner, wobei die Konzentration dieser Konservierungsmittel und Antioxidantien, die nachfolgend noch zusammengefaßt als sonstige Zusätze bezeichnet sind, insbesondere zwischen 0 Gew.% und 10 Gew.%, bezogen auf die anwendungsfertige Zusammensetzung, variiert.

Ist in bevorzugten Ausführungsformen der erfindungsgemäßen Zusammensetzung ein Verdickungsmittel oder ein Gelbildner vorhanden, so bietet es sich hierbei an, als Gelbildner bzw. als Verdickungsmittel ein natürliches und/oder synthetisches Kolloid und/oder ein natürliches und/oder synthetisches Hydrokolloid auszuwählen, wobei es sehr wohl möglich ist, daß die erfindungsgemäße Zusammensetzung ein Gemisch aus natürlichem und synthetischem Verdickungsmittel bzw. aus natürlichem und synthetischem Gelbildner enthält. Die Konzentration dieser Kolloide bzw. Hydrokolloide variiert üblicherweise zwischen 0.1 Gew.% und 5 Gew.%, jeweils bezogen auf die anwendungsfertige Zusammensetzung.

Als Beispiel geeigneter Gelbildner bzw. Verdickungsmittel sind insbesondere die an sich bekannten Stärkeether, Stärkeester, Celluloseether oder Celluloseester oder aber auch die Derivate der Acrylsäure und/oder Derivate der Acrylsäuresalze, insbesondere oligomere und polymere Acrylsäure bzw. Acrylsäuresalze oder Derivate davon zu nennen.

Bevorzugte Ausführungsformen der erfindungsgemäßen Zusammensetzung weisen als Trägersubstanz jeweils zwischen 0,5 Gew.% und 7 Gew.% eines hydrierten Phosphatidylcholins, zwischen 0,01 Gew.% und 5 Gew.% des Wirkstoffes und zwischen 5 Gew.% und 96 Gew.% Wasser als hydrophile Flüssigkeit auf, wobei dann diese bevorzugten Ausführungsformen desweiteren zwischen 0,5 Gew.% und 10 Gew.% Cupuacu Butter, zwischen 0,5 Gew.% und 15 Gew.% Sheabutter, zwischen 0,001 Gew.% und 3 Gew.% Ceramide, vorzugsweise Ceramide-1, Ceramide-3, Ceramide-6 und/oder Ceramide-7, zwischen 0,1 Gew.% und 5 Gew.% des Kolloids oder Hydrokolloids, zwischen 2 Gew.% und 42 Gew.% des zuvor beschriebenen Öls und/oder des zuvor beschriebenen Ölanteils, sowie zwischen 0 Gew.% und 10 Gew.% sonstiger Zusätze enthalten.

Bezüglich des pH-Wertes, die die erfindungsgemäße Zusammensetzung aufweist, ist insbesondere festzuhalten, daß hier ein pH-Wert ausgewählt wird, der vorzugsweise zwischen 4,0 und 7,6 und insbesondere zwischen 4,8 und 7,2, variiert.

Wie bereits vorstehend mehrfach herausgestellt wurde, ist ein wesentliches Kriterium der erfindungsgemäßen Zusammensetzung, daß diese die zuvor beschriebene spezielle Struktur aufweist und das desweiteren der mindestens eine Wirkstoff so zwischen der Doppelmembranschicht und der Schicht der inneren Phase verteilt ist, wie dies vorstehend quantifiziert ist. Insbesondere dann, wenn die erfindungsgemäße Zusammensetzung zwischen 10 Gew.% und 95 Gew.%, vorzugsweise zwischen 30 Gew.% und 95 Gew.% die Doppelmembranschicht enthält, wobei sich die zuvor angegebenen Konzentrationen auf das Gewicht der in der erfindungsgemäßen Zusammensetzung enthaltenen Trägersubstanz bezieht, weist eine derartige Ausgestaltung die zuvor bei der erfindungsgemäßen Zusammensetzung beschriebenen Vorteile in einem besonders hohen Ausmaß auf.

Es ist festzuhalten, daß die erfindungsgemäß Zusammensetzung insbesondere eine solche Struktur enthält, bei der jede Doppelmembranschicht eine Dicke zwischen 4 nm und 20 nm, insbesondere zwischen 4 mm und 8 nm, aufweist, wobei desweiteren vorzugsweise die Schichtdicke der inneren Phase, die zwischen benachbarten Doppelmembranschichten angeordnet ist, zwischen 2 nm und 10 nm variiert.

Wie bereits vorstehend wiederholt dargelegt ist, kann die erfindungsgemäße Zusammensetzung in jeder geeigneten Form appliziert werden, sei es beispielsweise als Creme, Salbe, Gel, Lotion oder als Zusatz zu einem Bad.

Besonders geeignet ist es jedoch, wenn die erfindungsgemäße Zusammensetzung als cremeartige oder gelartige Zusammensetzung vorliegt und eine Viskosität bei 20 °C zwischen 2.000 mPas und 40.000 mPas, vorzugsweise zwischen 12.000 mPas und 25.000 mPas, besitzt.

Eine besonders geeignete Ausgestaltung der erfindungsgemäßen Zusammensetzung sieht vor, daß hierbei die Zusammensetzung eine solche Struktur enthält, die zwischen 2 und 15 sandwichartig übereinander angeordnete lamellare Doppelmembranschichten umfaßt.

Der in der vorliegenden Anmeldung wiederholt verwendete Begriff "und/oder" deckt sowohl additiv als auch alternativ die so verknüpften einzelnen Elemente einer Aufzählung ab, so daß diese Elemente wahlweise mit "und" bzw. mit "oder" verknüpft zu verstehen sind. Desweiteren umfassen die im Singular verwendeten Begriffe selbstverständlich auch den Plural.

Die vorliegende Anmeldung wird durch die folgenden Aspekte näher beschrieben:
1. Topisch zu applizierende kosmetische oder pharmazeutische Zusammensetzung, die eine hydrophile äußere Phase, mindestens einen kosmetischen und/oder pharmazeutischen Wirkstoff sowie mindestens eine Trägersubstanz für den Wirkstoff aufweist und die Trägersubstanz solche Strukturen ausbildet, die mindestens zwei, sandwichartig übereinander angeordnete lamellare Doppelmembranschichten umfaßt, wobei zwischen benachbarten parallel zueinander ausgerichteten Doppelmembranschichten jeweils eine Schicht einer inneren Phase angeordnet ist, dadurch gekennzeichnet, daß der Wirkstoff in der Doppelmembranschicht und in der Schicht der inneren Phase verteilt ist, derart, daß die Schicht der innere Phase den Wirkstoff in einem Konzentrationsbereich zwischen 2 Gew.% und 98 Gew.% und die Doppelmembranschicht den Wirkstoff in einer Konzentration zwischen 98 Gew.% und 2 Gew.%, jeweils bezogen auf die Gesamtkonzentration an Wirkstoff, enthalten, und daß die äußere Phase keinen oder nahezu keinen Wirkstoff aufweist.
2. Zusammensetzung nach Aspekt 1, dadurch gekennzeichnet, daß die innere Phase den Wirkstoff in einem Konzentrationsbereich zwischen 15 Gew.% und 85 Gew.%, vorzugsweise in einem Konzentrationsbereich zwischen 25 Gew.% und 75 Gew.%, und die Doppelmembranschicht den Wirkstoff in einem Konzentrationsbereich zwischen 85 Gew.% und 15 Gew.%, vorzugsweise in einem Konzentrationsbereich zwischen 75 Gew.% und 25 Gew.%, jeweils bezogen auf die Gesamtkonzentration an Wirkstoff, enthalten.
3. Zusammensetzung nach Aspekt 1 oder 2, dadurch gekennzeichnet, daß die Konzentration des Wirkstoffes in der inneren Phase und der Doppelmembranschicht unterschiedlich sind.
4. Zusammensetzung nach einem der vorangehenden Aspekte, dadurch gekennzeichnet, daß die äußere Phase den Wirkstoff in einer Konzentration von bis zu maximal 5 Gew.%, bezogen auf die Gesamtkonzentration an Wirkstoff, aufweist.
5. Zusammensetzung nach Aspekt 4, dadurch gekennzeichnet, daß die äußere Phase den Wirkstoff in einer Konzentration zwischen 0 Gew.% und 2 Gew.%, bezogen auf die Gesamtkonzentration an Wirkstoff, aufweist.
6. Zusammensetzung nach einem der vorangehenden Aspekte, dadurch gekennzeichnet, daß die Zusammensetzung einen hydrophoben Wirkstoff enthält und daß der Wirkstoff überwiegend, vorzugsweise zu mindestens 70 Gew.%, bezogen auf die Gesamtkonzentration an Wirkstoff, innerhalb der Doppelmembranschicht angeordnet ist.
7. Zusammensetzung nach Aspekt 6, dadurch gekennzeichnet, daß innerhalb der Doppelmembranschicht der hydrophobe Wirkstoff in einer Konzentration zwischen 80 Gew.% und 90 Gew.%, bezogen auf die Gesamtkonzentration an Wirkstoff, eingelagert ist.
8. Zusammensetzung nach einem der vorangehenden Aspekte, dadurch gekennzeichnet, daß die Zusammensetzung einen hydrophilen Wirkstoff enthält und daß der Wirkstoff überwiegend, vorzugsweise zu mindestens 70 Gew.%, bezogen auf die Gesamtkonzentration an Wirkstoff, innerhalb der inneren Phase angeordnet ist.
9. Zusammensetzung nach Aspekt 6, dadurch gekennzeichnet, daß innerhalb der inneren Phase der hydrophile Wirkstoff in einer Konzentration zwischen 80 Gew.% und 90 Gew.%, bezogen auf die Gesamtkonzentration an Wirkstoff, eingelagert ist.
10. Zusammensetzung nach einem der vorangehenden Aspekte, dadurch gekennzeichnet, daß der Wirkstoff innerhalb der Zusammensetzung mittels einer lipophilen Verbindung an oder in der Doppelmembranschicht verankert ist.
11. Zusammensetzung nach Aspekt 10, dadurch gekennzeichnet, daß die lipophile Verbindung in der Doppelmembranschicht eingelagert und der hiermit verankerte Wirkstoff innerhalb der inneren Phase angeordnet ist.
12. Zusammensetzung nach Aspekt 10 oder 11, dadurch gekennzeichnet, daß die lipophile Verbindung den Wirkstoff über zwischenmolekulare Wechselwirkungen, insbesondere über Wasserstoffbrückenbindung oder über Van-der-Waals-Kräfte, fixiert.
13. Zusammensetzung nach einem der vorangehenden Aspekte , dadurch gekennzeichnet, daß die innere Phase und die äußere Phase identisch sind.
14. Zusammensetzung nach einem der vorangehenden Aspekte , dadurch gekennzeichnet, daß die innere Phase und/oder die äußere Phase Flüssigkeiten sind.
15. Zusammensetzung nach Aspekt 14, dadurch gekennzeichnet, daß innere Phase und die äußere Phase jeweils eine Flüssigkeit, insbesondere Wasser, sind.
16. Zusammensetzung nach einem der vorangehenden Aspekte , dadurch gekennzeichnet, daß die Zusammensetzung als Wirkstoff mindestens einen lokal oder systemisch wirkenden Wirkstoff enthält.
17. Zusammensetzung nach Aspekt 16, dadurch gekennzeichnet, daß der Wirkstoff ein pharmazeutischer Wirkstoff ist und aus der Gruppe ausgewählt ist, die Analgetika, Antirheumatika, Antiallergika, Antibiotika, Antimykotika, Antiphlogistika, Balneotherapeutika, corticoide Wirkstoffe, Antiseptika, durchblutungsfördernde Wirkstoffe, Sedativa, Anästhetika, Spasmolytika und Wundbehandlungsmittel, jeweils allein oder in Mischung, umfaßt.
18. Zusammensetzung nach einem der vorangehenden Aspekte, dadurch gekennzeichnet, daß der Wirkstoff ein kosmetischer Wirkstoff ist und aus der Gruppe ausgewählt ist, die Öle, Fette, Wachse, Antioxidantien, Peptide, Proteine, Aminosäuren, Derivate der Aminosäuren, Lichtschutzfilter, Bräunungsmittel, Vitamine, Pro-Vitamine, Fruchtsäuren, Feuchthaltesubstanzen, Pflanzenteile und Pflanzenextrakte, Harnstoff, Glucane, Glucanderivate, organische Metallverbindungen und anorganische Metallverbindungen umfaßt.
19. Zusammensetzung nach Aspekt 18, dadurch gekennzeichnet, daß die Zusammensetzung als Öl Wiesenschaumkrautöl, Avocadoöl, Kokosnußöl, Jojobaöl, Weizenkeimöl, Macadamianußöl, Aprikosenkernöl, Hanföl, Leinsamenöl, Sesamöl, Sonnenblumenöl, Erdnußöl, Rosmarienöl, Kamillenöl, Salbeiöl, Calendulaöl, Lavendelöl, Johanniskrautöl, Melissenöl, Sanddornöl, Teebaumöl, Zedernholzöl, Zypressenöl, Nachtkerzenöl, Johannisbeerkernöl, Borretschöl, Hagebuttenöl, Sojaöl, Fischöl, Mandelöl, Olivenöl und/oder Bestandteile dieser Öle aufweist.
20. Zusammensetzung nach Aspekt 18 oder 19, dadurch gekennzeichnet, daß das Öl bzw. der Ölbestandteil in der Zusammensetzung in einer Konzentration zwischen 0,5 Gew.% und 40 Gew.%, bezogen auf die anwendungsfertige Zusammensetzung, vorhanden ist.
21. Zusammensetzung nach einem der vorangehenden Aspekte, dadurch gekennzeichnet, daß die Zusammensetzung als pflegende kosmetische Wirkstoffe Sheabutter, Ceramide, insbesondere Ceramide-3, Cupuacu-Butter, Squalan und/oder Triglyceride, insbesondere mittelkettige, gesättigte C₈-C₂₄-Triglyceride, enthält.
22. Zusammensetzung nach einem der vorangehenden Aspekte, dadurch gekennzeichnet, daß die Zusammensetzung einen anti-entzündlichen Wirkstoff enthält, der ausgewählt ist aus der Gruppe, die die Ursolsäure, Soja-Sterol, 18-beta-Glycyrrhetinsäure, Gamma-Oryzanol, Ferula-Säure, Avenanthramide und Derivate der zuvor genannten Wirkstoffe umfaßt.
23. Zusammensetzung nach einem der vorangehenden Aspekte, dadurch gekennzeichnet, daß die Zusammensetzung den Wirkstoff in einer Konzentration zwischen 0,01 Gew.% und 35 Gew.%, vorzugsweise zwischen 0,1 Gew.% und 15 Gew.%, bezogen auf die anwendungsfertige Zusammensetzung, aufweist.
24. Zusammensetzung nach einem der vorangehenden Aspekte, dadurch gekennzeichnet, daß die in der Zusammensetzung enthaltene Trägersubstanz aus der Gruppe ausgewählt ist, die Monoglyceride, Diglyceride, Triglyceride, bevorzugt destillierte mittelkettige Monoglyceride, Sphingolipide, Phosphatidylcholin, Phospholipide, Fettalkohole, Fettsäuren und Derivate der zuvor genannten Verbindungen umfaßt.
25. Zusammensetzung nach einem der vorangehenden Aspekte, dadurch gekennzeichnet, daß die Zusammensetzung als Trägersubstanz ein hydriertes Phospholipid, insbesondere ein hydriertes Phosphatidylcholin, enthält.
26. Zusammensetzung nach Aspekt 25, dadurch gekennzeichnet, daß das hydrierte Phospholipid wenigstens 60 Gew.% hydriertes Phosphatidylcholin aufweist.
27. Zusammensetzung nach einem der vorangehenden Aspekte, dadurch gekennzeichnet, daß die Zusammensetzung die Trägersubstanz in einer Konzentration zwischen 0,5 Gew.% und 30 Gew.%, vorzugsweise in einer Konzentration zwischen 0,7 Gew.% und 10 Gew.%, bezogen auf die anwendungsfertige Zusammensetzung, aufweist.
28. Zusammensetzung nach einem der Aspekte 25 bis 27, dadurch gekennzeichnet, daß das hydrierte Phospholipid eine Phasenübergangstemperatur über 30 °C und unter 70 °C besitzt.
29. Zusammensetzung nach einem der vorangehenden Aspekte, dadurch gekennzeichnet, daß die Zusammensetzung die äußere Phase und die innere Phase in einer Gesamtkonzentration zwischen 5 % und 90 %, bezogen auf das Gewicht der anwendungsfertigen Zusammensetzung, aufweist.
30. Zusammensetzung nach einem der vorangehenden Aspekte, dadurch gekennzeichnet, daß die Zusammensetzung desweiteren mindestens einen Alkohol, insbesondere einen mehrwertigen Alkohol, enthält.
31. Zusammensetzung nach Aspekt 30, dadurch gekennzeichnet, daß die Zusammensetzung als Alkohol Phenylethylalkohol, Pentylenglykol, Caprylyl Glykol, Decylen Glykol und/oder Glycerin aufweist .
32. Zusammensetzung einem der vorangehenden Aspekte , dadurch gekennzeichnet, daß die Zusammensetzung ein N-Acyl-Ethanolamin in einer Konzentration zwischen 0,01 Gew.% und 10 Gew.%, vorzugsweise zwischen 0,1 Gew.% und 3 Gew.%, bezogen auf die anwendungsfertige Zusammensetzung, aufweist.
33. Zusammensetzung nach Aspekt 32, dadurch gekennzeichnet, daß der Acylrest des N-Acyl-Ethanolamins ein C₁-C₂₄-Acylrest ist.
34. Zusammensetzung nach Aspekt 32 oder 33, dadurch gekennzeichnet, daß das N-Acyl-Ethanolamin aus der Gruppe ausgewählt ist, die N-Acetyl-Ethanolamin, N-Oleoyl-Ethanolamin, N-Linolenoyl-Ethanolamin, N-Cocoyl-Ethanolamin und N-Palmitoyl-Ethanolamin umfaßt.
35. Zusammensetzung nach einem der vorangehenden Aspekte, dadurch gekennzeichnet, daß die Zusammensetzung desweiteren mindestens ein Konservierungsmittel, ein Antioxidanz, ein Verdickungsmittel und/oder einen Gelbildner aufweist.
36. Zusammensetzung nach Aspekt 35, dadurch gekennzeichnet, daß die Zusammensetzung als Verdickungsmittel bzw. als Gelbildner ein natürliches Kolloid oder ein natürliches Hydrokolloid und/oder ein synthetisches Kolloid oder ein synthetisches Hydrokolloid aufweist.
37. Zusammensetzung nach einem der vorangehenden Aspekte, dadurch gekennzeichnet, daß die Zusammensetzung jeweils zwischen
   0,5 Gew.% und 7 Gew.% hydriertes Phosphatidylcholin,
   0,5 Gew.% und 10 Gew.% Cupuacu Butter
   0,5 Gew.% und 15 Gew.% Sheabutter
   0,001 Gew.% und 3 Gew.% Ceramide
   0,1 Gew.% und 5 Gew.% des Kolloids oder Hydrokolloids
   2 Gew.% und 42 Gew.% des Öls oder des Ölbestandteils
   0,01 Gew.% und 5 Gew.% des Wirkstoffs
   0 Gew.% und 10 Gew.% sonstiger Zusätze und
   5 Gew.% und 96 Gew.% Wasser
   aufweist.
38. Zusammensetzung nach einem der vorangehenden Aspekte, dadurch gekennzeichnet, daß die Zusammensetzung als topisch applizierbares Zusammensetzung formuliert ist und eine Viskosität bei 20 °C zwischen 2.000 mPas und 40.000 mPas, vorzugsweise zwischen 12.000 mPas und 25.000 mPas, aufweist.
39. Zusammensetzung nach einem der vorangehenden Aspekte, dadurch gekennzeichnet, daß die Zusammensetzung einen pH-Wert zwischen 4,0 und 7,6 besitzt.
40. Zusammensetzung nach einem der vorangehenden Aspekte, dadurch gekennzeichnet, daß die Zusammensetzung die Doppelmembranschicht in einer Konzentration zwischen 10 % und 95 %, vorzugsweise zwischen 30 % und 95 %, bezogen auf das Gewicht der in der Zusammensetzung enthaltenen Trägersubstanz aufweist.
41. Zusammensetzung nach einem der vorangehenden Aspekte, dadurch gekennzeichnet, daß die Zusammensetzung eine Struktur enthält, die zwischen 2 und 15 sandwichartig übereinander angeordnete lamellare Doppelmembranschichten umfaßt.
42. Zusammensetzung nach einem der vorangehenden Aspekte, dadurch gekennzeichnet, daß jede einzelne Doppelmembrane eine Dicke zwischen 4 nm und 20 nm, vorzugsweise zwischen 4 nm und 8 nm, besitzt.
43. Zusammensetzung nach einem der vorangehenden Aspekte, dadurch gekennzeichnet, daß die Schicht der zwischen benachbarten Doppelmembranschichten angeordneten inneren Phase eine Dicke zwischen 2 nm und 10 nm aufweist.

Vorteilhafte Weiterbildungen der erfindungsgemäßen Zusammensetzung sind in den Unteransprüchen angegeben.

Die erfindungsgemäße Zusammensetzung wird nachfolgend anhand von vier Ausführungsbeispielen in Verbindung mit der Zeichnung erläutert. Es zeigen:
- Figur 1: eine schematische Darstellung einer ersten Ausführungsform der zuvor beschriebenen speziellen Struktur;
- Figur 2: eine schematische Darstellung einer zweiten Ausführungsform der zuvor beschriebenen speziellen Struktur; und
- Figur 3: eine schematische Darstellung einer dritten Ausführungsform der zuvor beschriebenen speziellen Struktur.

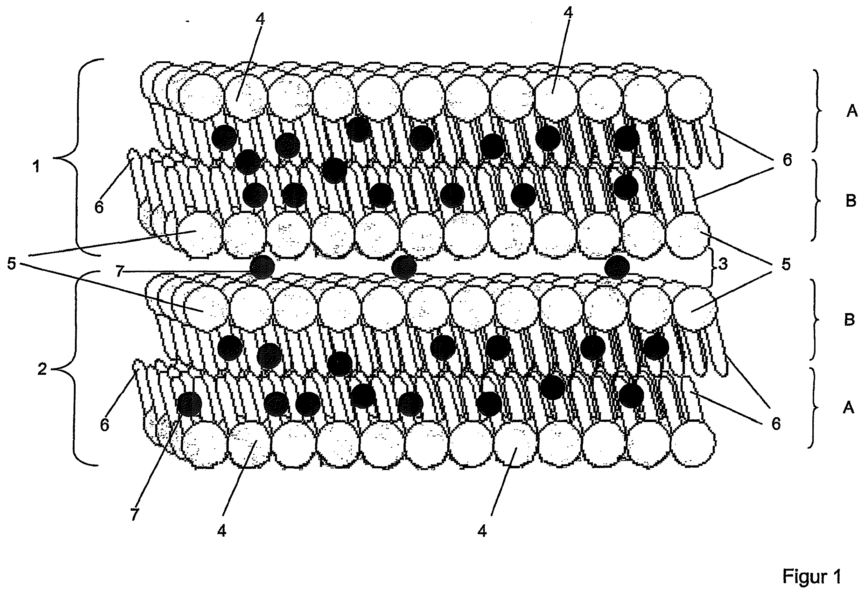

In den Figuren 1 bis 3 sind die selben Elemente mit den selben Bezugszeichen versehen.

Die Figuren 1 bis 3 bilden unterschiedliche Ausführungsformen der Strukturen, wie sie in der erfindungsgemäßen Zusammensetzung vorkommen und für diese wesentlich sind, ab.

Alle in den Figuren 1 bis 3 schematisch gezeigten Strukturen weisen gemeinsam eine planare erste Doppelmembranschicht 1 und eine planare zweite Doppelmembranschicht 2 auf, wobei die Doppelmembranschichten 1 und 2 sandwichartig die planare Schicht einer inneren Phase 3 einschließen.

Jede Doppelmembranschicht 1 bzw. 2 besteht aus zwei Lagen A und B der Trägersubstanz, wobei innerhalb der beiden Lagen A bzw. B die einzelnen Moleküle der Trägersubstanz so ausgerichtet sind, daß die äußeren hydrophilen Reste 4 der oberen Lage A einer jeden Doppelmembranschicht 1 bzw. 2 jeweils nach außen zur äußeren hydrophilen Phase, die die jeweilige Struktur komplett umgibt, ausgerichtet sind, während die inneren hydrophilen Reste 5 der unteren Lage B nach innen hin zur Schicht der inneren Phase 3 weisen. Dies hat zur Folge, daß innerhalb einer jeden Lage A bzw. B die lipophilen Reste 6 einer jeden Doppelmembranschicht 1 bzw. 2 zueinander ausgerichtet sind. Dementsprechend kommen bei den in den Figuren 1 bis 3 gezeigten Strukturen nur die äußeren hydrophilen Reste 4 in Kontakt mit der äußeren Phase, während die inneren hydrophilen Reste 5 einer jeden Doppelmembranschicht 1 bzw. 2 ausschließlich mit der Schicht der inneren Phase 3 in Kontakt gelangen.

Mit 7 sind jeweils dunkel abgebildete Wirkstoffmoleküle bzw. dunkel abgebildete Wirkstoffaggregate bezeichnet, wobei sich die Wirkstoffmoleküle von den Wirkstoffaggregaten dadurch unterscheiden, daß die Wirkstoffaggregate Zusammenschlüsse von Wirkstoffmolekülen darstellen, was nachfolgend kurz als Wirkstoff 7 bezeichnet wird.

Die schematischen Abbildungen gemäß der Figuren 1 bis 3 unterscheiden sich dahingehend voneinander, daß der Wirkstoff 7 unterschiedlich zwischen den Schichten 1 bis 3 verteilt ist.

In Figur 1 ist die überwiegende Menge des Wirkstoffes 7 in der Doppelmembranschicht 1 bzw. 2 verteilt und dort zwischen den hydrophoben Resten 6 eingelagert, während die Schicht der inneren Phase 3 eine relativ geringe Konzentration an Wirkstoff 7 aufweist. Eine derartige Struktur weist insbesondere eine solche Zusammensetzung auf, die einen überwiegenden hydrophoben Wirkstoff enthält.

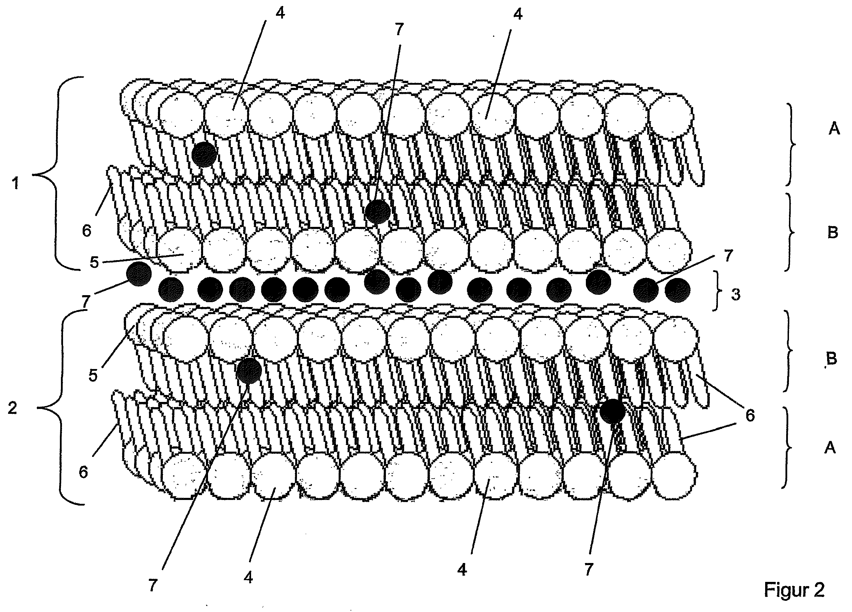

In Figur 2 ist die überwiegende Menge des Wirkstoffes 7 in die Schicht der inneren Phase 3 eingelagert, während die Doppelmembranschicht 1 bzw. 2 eine relativ geringe Konzentration an Wirkstoff 7 aufweist, der zwischen den hydrophoben Resten 6, vorzugsweise in der unmittelbaren Nähe der hydrophilen Reste 4 oder 5, eingelagert ist. Eine derartige Struktur weist insbesondere eine solche Zusammensetzung auf, die einen überwiegenden hydrophilen Wirkstoff enthält.

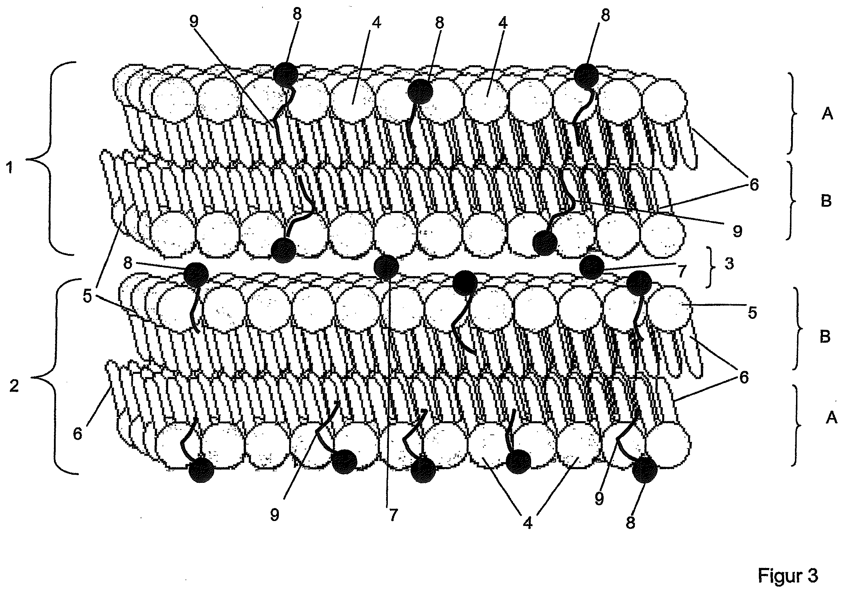

Die Figur 3 bildet eine solche Struktur ab, bei der die innere Phase 3 eine relativ geringe Konzentration an Wirkstoff 7 enthält. Hierbei handelt es sich um den ursprünglichen Wirkstoff. Desweiteren bildet die Figur 3 schematisch solche Wirkstoffe 8 ab, bei denen es sich ebenfalls um Moleküle bzw. Aggregate handeln kann, die mittels lipophiler Verbindungen, die in der Figur 3 als dunkle, gebogene Striche 9 schematisch abgebildet sind, innerhalb der Doppelmembranschicht 1 bzw. 2 verankert sind. Eine derartige Struktur weist insbesondere eine solche Zusammensetzung auf, die überwiegend einen hydrophilen Wirkstoff enthält, wobei ein Teil dieses Wirkstoffes mit den lipophilen Verbindungen (Anker-gruppe) unter Ausbildung von zwischenmolekularen Wechselwirkungen zwischen lipophiler Verbindung und Wirkstoff umgesetzt wird, während der verbleibende Teil einen entsprechend nicht umgesetzten Wirkstoff 7 enthält. Den Grad der Umsetzung kann durch Abstimmung der stöchiometrischen Verhältnisse von Wirkstoff und lipophiler Verbindung bestimmt werden. Diese Möglichkeit besteht nicht nur für hydrophile Wirkstoffe sondern auch für amphiphile Wirkstoffe.

### Ausführungsbeispiel A

### Herstellung eines Retinol als Wirkstoff enthaltenen Konzentrates

Es wurde ein Retinol als Wirkstoff enthaltenes Konzentrat aus folgenden Inhaltsstoffen hergestellt:

| Inhaltsstoffe der Phase 1: | |
|---|---|
| 6 | Gew.% hydriertes Phosphatidylcholin |
| 3 | Gew.% Caprylic/Capric Triglyceride |
| 3 | Gew.% Glycerin |
| 5 | Gew.% Pentylene Glycol |
| | |

| Inhaltsstoff der Phase 2: | |
|---|---|
| 10 | Gew.% Retinol |
| | |

| Inhaltsstoff der Phase 3: | |
|---|---|
| 73 | Gew.% Wasser |

Die Inhaltsstoffe der Phase 1 wurden unter gleichmäßigem Rühren auf 80 °C erwärmt. Anschließend wurde der Inhaltsstoff der Phase 3 auf 75 °C erwärmt. Bei 80 °C wurde der Inhaltsstoff der Phase 2 zu den Inhaltsstoffen der Phase 1 gegeben und gleichmäßig miteinander verrührt. Anschließend wurde der Inhaltsstoffe der Phase 3 zu den miteinander verrührten Inhaltsstoffen der Phase 1 und 2 gegeben, so daß die so miteinander vermischten Inhaltsstoffe aller Phasen bei 20.000 U/min mittels Ultra Turrax homogenisiert worden sind. Die entstandene Präemulsion wurde mittels eines Hochdruckhomogenisators unter folgenden Bedingungen feinstdispergiert: 2 - 5 Zyklen bei 800 bar. Nach Abkühlung des feindispergierten Gemisches unter gleichmäßigem Rühren auf 30 °C wurde für 2 Minuten bei 20.000 U/min mittels Ultra Turrax nochmals homogenisiert.

Retinol gilt in der Kosmetik als schwer zu stabilisierender Wirkstoff, der einem ständigen oxidativen Abbauprozeß unterliegt. Klassische Stabilisierungsverfahren wie der Zusatz von Antioxidantien, Verkapselung in Liposomen oder Cyclodextrinen stoßen immer wieder an Ihre Grenzen, da sie entweder nicht die gewünschte Wirkstoffkonzentration erreichen oder nicht die erforderliche Stabilität aufweisen.

In einem Vergleichsversuch konnte erstaunlicherweise festgestellt werden, daß die Retinol-Stabilität durch die beschriebene Zusammensetzung deutlich erhöht wurde.

Für diesen Versuch wurde eine liposomale, Retinol in der vorstehend angegebenen Konzentration enthaltende Formulierung mit dem zuvor beschriebenen Konzentrat verglichen.

Zu diesem Zweck wurde die jeweilige Probe einer Lichtbestrahlung (1.4 mW/cm2 über 20 Minuten) als Streßparameter ausgesetzt. Die Bestrahlung fand in Quarzglaskammern mit einem wassergefülltem Deckel (Fa. Heraeus Quarzglas GmbH) statt, der dazu diente, die Infrarotenergie zu absorbieren. Dieses war erforderlich, um die Verdampfung des Lösungsmittels in der jeweiligen Probe während der Bestrahlung zu vermeiden. Die nach der Bestrahlung verbleibende Retinol Konzentration wurde durch Trennung mittels Hochdruckflüssigkeitschromatographie (RP-18 Säule, mobile Phase bestehend aus einem Gemisch Methanol -n-Hexan 72:28 (vol./vol.)) und anschließender Detektion der UV Absorption bei 324, 292 and 276 nm durchgeführt. Die Bestrahlung wurde für jede Probe dreimal durchgeführt, um so die Reproduzierbarkeit zu gewährleisten.

Die liposomale Formulierung erlaubte lediglich eine 30 %ige Stabilisierung des Retinols, so daß durch die Bestrahlung 70 Gew.% des ursprünglichen Retinols abgebaut wurden, während die Retinol Konzentration nach der Bestrahlung des Konzentrates bei 70 % lag, so daß hierbei nur 30 Gew.% des ursprünglich eingesetzten Wirkstoffes Retinol abgebaut wurde.

### Ausführungsbeispiel B

### Herstellung eines Boswellia als Wirkstoff enthaltenen Konzentrates

| Inhaltsstoffe der Phase 1: | |
|---|---|
| 7,5 | Gew.% hydriertes Phosphatidylcholin |
| 3 | Gew.% Caprylic/Capric Triglyceride |
| 3 | Gew.% Glycerin |
| 5 | Gew.% Hexylene Glycol |
| 3 | Gew.% Meadow Foam Seed Oil |
| 5 | Gew.% Boswellia Serrata extract |
| | |

| Inhaltsstoff der Phase 2: | |
|---|---|
| 73,5 | Gew.% Wasser |

Die Inhaltsstoffe der Phase 1 wurden unter gleichmäßigem Rühren auf 85 °C erwärmt. Anschließend wurde der Inhaltsstoffe der Phase 2 ebenfalls auf 85 °C erwärmt und bei dieser Temperatur zur Phase 1 gegeben und hiernach die vermischten Phasen gleichmäßig verrührt und bei 24.000 U/min mittels Ultra Turrax homogenisiert. Die entstandene Präemulsion wurde mittels eines Hochdruckhomogenisators während 4 - 6 Zyklen bei 750 bar feinstdispergiert. Nach Abkühlen auf 35 °C wurde die Feindispersion nochmals für 2 Minuten bei 20.000 U/min mittels Ultra Turrax homogenisiert. Das so hergestellte Konzentrat wurde unter Rühren auf 30 °C abgekühlt.

Boswellia Serrata extract ist aufgrund seiner antiinflammatorischen Eigenschaften von großem Interesse als Wirkstoff für die kosmetische und pharmazeutische Industrie. Aufgrund seiner harzartigen Eigenschaften gilt Boswellia Serrata extract jedoch als schwer zu stabilisierendes Molekül, da es die Emulsionsbildung erheblich beeinträchtigt und von daher nur in geringen Konzentrationen in kosmetischen oder pharmazeutischen Produkten eingesetzt wird.

Durch einen Versuch, der das zuvor beschriebene Konzentrat mit dem dort genannten Wirkstoff in der dort aufgeführten Konzentration mit einer herkömmlichen Boswellia Serrata extract Emulsion, die in ihrer Konzentration an Wirkstoff dem Konzentrat entsprach, vergleicht, konnte festgestellt werden, daß nur das Konzentrat eine stabile und hochkonzentrierte Darreichungsform für den Wirkstoff zur Verfügung stellt.

Durch makroskopische und mikroskopische Analyse (Mikroskop: Olympus CH2 Modell CHT) wurde nachgewiesen, daß die nach Lagerung bei der herkömmlichen Emulsion auftretende typischen Destabilisierungserscheinungen bei dem Konzentrat nicht auftreten. Insbesondere zeigte das Konzentrat im Gegensatz zur herkömmlichen Emulsion makroskopisch keine Entmischung (Phasentrennung) mit anschließender deutlich erkennbarer Ölseparation. Desweiteren wurden sowohl das Konzentrat als auch die herkömmliche Emulsion mikroskopisch bei einer 400fachen Vergrößerung hinsichtlich der darin enthaltenen kristallinen Strukturen und des Tröpfchengrößenwachstums untersucht. Hierbei konnte festgestellt werden, daß bei der herkömmlichen Emulsion innerhalb der ersten 3 Tage nach Herstellung (Lagerung bei 23 ± 2°C) ein deutliches Teilchengrößenwachstum und eine morphologische Veränderung der Lipidtröpfchenform hin zu amorphen, unsymetrischen Strukturen auftraten, was von der Fachwelt als sicheres Zeichen für eine beginnende, auch makroskopisch festzustellende Phasentrennung bewertet wird.

Im Gegensatz hierzu konnte bei dem Konzentrat weder makroskopisch noch mikroskopisch eine Veränderung festgestellt werden, so daß das Konzentrat auch über Monate hinaus stabil und unverändert blieb.

### Ausführungsbeispiel C

### Herstellung eines Prolin als Wirkstoff enthaltenen Konzentrates

| Inhaltsstoffe der Phase 1: | |
|---|---|
| 6 | Gew.% hydriertes Phosphatidylcholin |
| 7 | Gew.% Jojoba oil |
| 3 | Gew.% Glycerin |
| 5 | Gew.% Pentylene Glycol |
| 3 | Gew.% Butyrospermum Parkii |
| | |

| Inhaltsstoff der Phase 2: | |
|---|---|
| 15 | Gew.% Proline |
| | |

| Inhaltsstoff der Phase 3: | |
|---|---|
| 61 | Gew.% Wasser |

Die Inhaltsstoffe der Phase 1 wurden unter gleichmäßigem Rühren auf 80 °C erwärmt. Bei 80 °C wurden der Inhaltsstoff der Phase 2 zu der vermischten Phase 1 gegeben und gleichmäßig verrührt. Hiernach wurde die auf 75 °C erwärmte Phase 3 zu den miteinander vermischten Inhaltsstoffen der Phasen 1 und 2 gegeben und bei 20.000 U/min mittels Ultra Turrax homogenisiert. Die so hergestellte Präemulsion wurde mittels Hochdruckhomogenisators für 2 - 5 Zyklen bei 800 bar feinstdispergiert. Nach Abkühlen auf 30 °C unter gleichmäßigem Rühren wurde das so erstellte Gemisch für 2 Minuten bei 20.000 U/min mittels Ultra Turrax homogenisiert.

Bei diesem Ausführungsbeispiel dient das Prolin als Modellsubstanz für ein Osmoprotectants.

In einem Versuch, der einerseits eine herkömmliche Öl-in-Wasser-Emulsion, die die gleiche Konzentration an Prolin enthielt und andererseits das zuvor beschriebene Konzentrat umfaßt, wurde vergleichend die in der Oberschicht der Haut vorhandene Prolin-Konzentration nach Applikation des jeweiligen Produktes bestimmt.

Nach Applikation der jeweiligen Probe und Ablauf einer 60 minütigen Verweilzeit wurden nach der Stripping Methode 10 Tesafilmabrisse von dem gleichen Hautareal genommen. Für den Vergleichsversuch wurde darauf geachtet, daß identisch große Hautareale mit identischen Mengen des Konzentrates bzw. der Öl-in-Wasser-Emulsion behandelt wurden. Die entsprechenden Tesafilmstreifen wurden mit jeweils einem 1 ml Methanol extrahiert. Die Konzentration an Prolin wurde mittels Hochdruckflüssigkeitschromatographie (CROWNPAK CR(+) Säule, mobile Phase bestehend aus einem HCLO4 Lösung, Vorsäulen Derivatisierung mit DABS-CL (CrestPak C18S Säule, mobile Phase: 8mM Sodium Dihydrogenphosphate Dihydrate in H2O with 4% DMF, Detektion der UV Absorption bei 280 nm) bestimmt, wobei alle Werte als Dreifachbestimmung ermittelt wurden.

Als Ergebnis ist festzuhalten, daß die in der Haut ermittelte Prolin-Konzentration nach Applikation des Konzentrates um 50 % höher lag als die Prolin-Konzentration, die nach Applikation der Öl-in-Wasser-Emulsion gemessen wurde.

### Ausführungsbeispiel D

### Herstellung eines Palmitoyl Pentapeptide-3 als Wirkstoff enthaltenen Konzentrates

| Inhaltsstoffe der Phase 1: | |
|---|---|
| 8 | Gew.% hydriertes Phosphatidylcholin |
| 11 | Gew.% Isopropyl Palmitate |
| 3 | Gew.% Glycerin |
| 10 | Gew.% Ethanol |
| | |

| Inhaltsstoff der Phase 2: | |
|---|---|
| 0,3 | Gew.% Palmitoyl Pentapeptide-3 |
| | |

| Inhaltsstoff der Phase 3: | |
|---|---|
| 67,7 | Gew.% Wasser |

Die Inhaltsstoffe der Phase 1 wurden unter gleichmäßigem Rühren auf 80 °C erwärmt. Bei 80 °C wurde der Inhaltsstoffe der Phase 2 zu der Phase 1 gegeben und gleichmäßig verrührt. Der auf 75 °C erwärmte Inhaltsstoff der Phase 3 wurde zu den Inhaltsstoffen der Phasen 1 und 2 gegeben und bei 20.000 U/min mittels Ultra Turrax homogenisiert. Die entstandene Präemulsion wurde mittels Hochdruckhomogenisators für 2 - 5 Zyklen bei 800 bar feinstdispergiert und anschließend unter gleichmäßigem Rühren auf 30 °C abgekühlt. Hieran schloß sich für 2 Minuten ein erneutes Homogenisieren bei 20.000 U/min mittels Ultra Turrax an.

Palmitoyl Pentapeptide-3 sind seit Jahren im Zentrum des dermatologisch - kosmetischen Interesses. Ähnlich wie Kupfer Peptide, stimulieren Palmitoyl Pentapeptide die Wundheilungsprozesse in den tieferen Schichten der Haut durch Produktion von Kollagen und Fibronektin. Dadurch wird der Hautalterung aktiv entgegengewirkt und Wundheilungsprozesse aktiv unterstützt, wobei die Wirkung häufig erst im Zeitrahmen von 4 bis 6 Wochen eintritt.

Nach der zuvor beschriebenen Methode wurde das zuvor beschriebene Konzentrat im Vergleich zu einer herkömmlichen Formulierung untersucht, wobei die zuvor beschriebenen Versuchsbedingungen angewendet wurden. Hiernach konnte festgestellt werden, daß die Penetrationsmenge an Palmitoyl Pentapeptide-3 bei Anwendung des Konzentrates im Vergleich zu der herkömmlichen Formulierung um 40 % höher lag.

Die nach den Ausführungsbeispielen A bis D hergestellten Kornzentrate können durch Verdünnen in einem Verhältnis zwischen 5 bis 50 Gew.% Konzentrat mit 95 bis 50 Gew.% Zusatzstoffen, so beispielsweise Wasser, Verdickungsmittel, Hydrogele oder weiteren kosmetischen Wirkstoffen, zu einem anwendungsfertigen Produkt verarbeitet werden.

### Ausführungsbeispiel E

### Herstellung einer Endformulierung mit dem Wirkstoff Hexapeptide-9

| Inhaltsstoffe der Phase 1: | |
|---|---|
| 2 | Gew.% hydriertes Phosphatidylcholin |
| 1 | Gew.% Hexapeptid-9 |
| 0,8 | Gew.% Butyrospermum Parkii |
| 1,5 | Gw.% Caprylic/Capric Triglyceride |
| 1 | Gew.% Squalane |
| | |

| Inhaltsstoffe der Phase 2: | |
|---|---|
| | |
| 1 | Gew.% Glycerin |
| 1,3 | Gew.% Pentylene Glycol |
| 19 | Gew.% Wasser |

| Inhaltsstoffe der Phase 3: | |
|---|---|
| 25 | Gew.% Caprylic/ Capric Triglyceride |
| 0,10 | Gew.% Carbomer |
| 0,10 | Gew.% Sodium Carbomer |
| 0,10 | Gew.% Xanthan Gum |
| | |

| Inhaltsstoffe der Phase 4: | |
|---|---|
| 3,5 | Gew.% Pentylene Glycol |
| 0,35 | Gew.% Hydroxyethylcellulose |
| ad 100,0 | Gew.% Wasser |

Die Inhaltsstoffe der Phase 1 wurden unter gleichmäßigem Rühren auf 85 °C erwärmt bis alle Wirkstoffe gelöst vorliegen. Ebenfalls wurde die Phase 2 in einem getrennten Gefäß auf 85 °C unter Rühren erwärmt. Anschließend wurde die Phase 2 zu der Phase 1 gegeben, kurz verrührt und hiernach mittels Ultra Turrax bei 24.000 U/min homogenisiert.

Die hieraus resultierende Präemulsion wurde mittels Hochdruckhomogenisators in 5 - 7 Zyklen, Druck 600 bar, feinstdispergiert. Die entstandene Dispersion wurde unter gleichmäßigem Rühren auf 30 °C abgekühlt.

Phase 3 und Phase 4 wurden in einem jeweils separaten Gefäß unter gleichmäßigem Rühren auf 30 °C erwärmt. Anschließend wurde Phase 4 zur Phase 3 gegeben und anschließend per Ultra Turrax homogenisiert (12.000 U/min). Unter leichtem Rührern wurde die entstandene Dispersion auf 30 °C abgekühlt. Dann wurde die hochviskose Dispersion aus den Phasen 1 und 2 zugegeben. Hiernach wurde die Mischung bei 30 °C per Ultra Turrax (12.000 U/min) homogenisiert bis eine gleichmäßige Struktur vorlag. Die so erstellte Endformulierung konnte direkt verwendet werden.

### Ausführungsbeispiel F

### Herstellung einer Endformulierung mit den UVB Filter Octocrylene und dem UVA Filter Butylmethoxydibenzoylmethane

| Inhaltsstoffe der Phase 1: | |
|---|---|
| 2,10 | Gew.% hydriertes Phosphatidylcholin |
| 3,00 | Gew,% Octocrylene |
| 2,50 | Gew.% Butylmethoxydibenzoylmethane |
| | |

| Inhaltsstoffe der Phase 2: | |
|---|---|
| 1,00 | Gew.% Glycerin |
| 1,30 | Gew.% Pentylene Glycol |
| 18,00 | Gew.% Wasser |
| 0,10 | Gew.% Caprylyl Glycol |
| | |

| Inhaltsstoffe der Phase 3: | |
|---|---|
| 22,00 | Gew.% Caprylic/ Capric Triglyceride |
| 0,10 | Gew.% Carbomer |
| 0,10 | Gew.% Sodium Carbomer |
| 0,10 | Gew.% Xanthan Gum |
| | |

| Inhaltsstoffe der Phase 4: | |
|---|---|
| 3,50 | Gew.% Pentylene Glycol |
| 0,35 | Gew.% Hydroxyethylcellulose |
| ad 100,0 | Gew.% Wasser |

Die Inhaltsstoffe der Phase 1 wurden unter gleichmäßigem Rühren auf 85 °C erwärmt bis alle Inhaltsstoffe gelöst vorliegen. Ebenfalls wurde die Phase 2 in einem getrennten Gefäß auf 85 °C unter Rühren erwärmt. Anschließend wurde die Phase 2 zu der Phase 1 gegeben und hiernach mittels Ultra Turrax bei 24.000 U/min so lange verrührt, bis eine homogene Mischung entstand. Die hieraus resultierende Präemulsion wurde mittels Hochdruckhomogenisators in 6 - 8 Zyklen, Druck 800 bar, feinstdispergiert. Die entstandene Dispersion wurde unter gleichmäßigem Rühren auf 30 °C abgekühlt.

Phase 3 und Phase 4 wurden in einem separaten Gefäß unter gleichmäßigem Rühren auf 30 °C erwärmt. Anschließend wurde Phase 4 zur Phase 3 gegeben und hiernach per Ultra Turrax (12.000 U/min) so lange verrührt, bis eine homogene Mischung entstand. Unter leichtem Rühren wurde die entstandene Dispersion auf 30 °C abgekühlt. Dann wurde die hochviskose Dispersion aus den Phasen 1 und 2 zugegeben. Anschließend wurde die Mischung bei 30 °C per Ultra Turrax (12.000 U/min) so lange homogenisiert, bis eine gleichmäßige Struktur vorlag. Die so erstellte Endformulierung konnte direkt verwendet werden.

### Ausführungebeispiel G

### Herstellung einer Endformulierung mit dem Wirkstoff Hypericin zur Behandlung von Herpes

| Inhaltsstoffe der Phase 1: | |
|---|---|
| 1,50 | Gew. % hydriertes Phosphatidylcholin |
| 0,05 | Gew. % Hypericin |
| 3,00 | Gew. % Butyrospermum Parkii |
| 0,25 | Gew. % Squalane |
| | |

| Inhaltsstoffe der Phase 2: | |
|---|---|
| 1,00 | Gew. % Glycerin |
| 3,00 | Gew. % Ethanol |
| 19,00 | Gew. % Wasser |
| | |

| Inhaltsstoffe der Phase 3: | |
|---|---|
| 10,00 | Gew. % Oleo europeae oil |
| 14,00 | Gew. % Butyrospermum Parkii |
| 0,10 | Gew. % Carbomer |
| 0,10 | Gew. % Sodium Carbomer |

| Inhaltsstoffe der Phase 4: | |
|---|---|
| 10,00 | Gew. % Ethanol |
| 8,00 | Gew. % Sorbitol |
| 0,25 | Gew. % Hydroxyethylcellulose |
| ad 100,0 | Gew. % Wasser |
| | |

| Inhaltsstoffe der Phase 5: | |
|---|---|
| 0,20 | Aroma Vanilla |

Die Inhaltsstoffe der Phase 1 wurden unter gleichmäßigem Rühren auf 85 °C erwärmt bis alle Inhaltsstoffe gelöst vorlagen. Ebenfalls wurde die Phase 2 in einem getrennten Gefäß auf 85 °C unter Rühren erwärmt. Hiernach wurde die homogene Phase 2 zu der Phase 1 gegeben, kurz verrührt und danach mittels Ultra Turrax bei 24.000 U/min homogenisiert. Die hieraus resultierende Präemulsion wurde mittels Hochdruckhomogenisators in 5 - 7 Zyklen, Druck 600 bar, feinstdispergiert. Die entstandene Dispersion wurde unter gleichmäßigem Rühren auf 30 °C abgekühlt.

Die Phasen 3 und 4 wurden jeweils in einem separaten Gefäß unter gleichmäßigem Rühren auf 50 °C erwärmt. Anschließend wurde die Phase 4 zur Phase 3 gegeben und hiernach per Ultra Turrax homogenisiert (12.000 U/min). Unter leichtem Rühren wurde die entstandene Dispersion auf 30 °C abgekühlt. Danach wurde die Phase 5 zu der Mischung gegeben und wiederum per Ultra Turrax kurz homogenisiert (10.000 U/min) bis der Aromastoff gleichmäßig eingearbeitet war. Dann wurde die hochviskose Dispersion aus den Phasen 1 und 2 zugesetzt. Hiernach wurde die Mischung bei 30 °C per Ultra Turrax (12.000 U/min) so lange homogenisiert, bis eine gleichmäßige Struktur vorlag. Die so erstellte Endformulierung konnte direkt verwendet werden.

### Ausführungsbeispiel H

### Herstellung einer Endformulierung mit dem Wirkstoff Panthenyl Triacetate zur Behandlung der Beschwerde bei trockener Nasenschleimhaut

| Inhaltsstoffe der Phase 1: | |
|---|---|
| 1,50 | Gew.% hydriertes Phosphatidylcholin |
| 1,00 | Gew.% Panthenyl triacetate |
| 0,80 | Gew.% Butyrospermum Parkii |
| 1,50 | Gew.% Caprylic/Capric Triglyceride |
| 0,200 | Gew.% Squalane |
| | |

| Inhaltsstoffe der Phase 2: | |
|---|---|
| 1,00 | Gew.% Glycerin |
| 1,30 | Gew.% Pentylene Glycol |
| 17,00 | Gew. % Wasser |
| | |

| Inhaltsstoffe der Phase 3: | |
|---|---|
| 10,00 | Gew.% Caprylic/ Capric Triglyceride |
| 8,00 | Gew.% Butyrospermum Parkii |
| 0,10 | Gew.% Carbomer |
| 0,10 | Gew.% Sodium Carbomer |
| 0,10 | Gew.% Xanthan Gum |
| | |

| Inhaltsstoffe der Phase 4: | |
|---|---|
| 3,50 | Gew.% Pentylene Glycol |
| 0,30 | Gew.% Sodium Hyaluronate |
| ad 100,0 | Gew.% Wasser |

Die Inhaltsstoffe der Phase 1 wurden unter gleichmäßigem Rühren auf 85 °C erwärmt bis alle Wirkstoffe gelöst vorlagen. Ebenfalls wurde die Phase 2 in einem getrennten Gefäß auf 85 °C unter Rühren erwärmt. Anschließend wurde die Phase 2 zu der Phase 1 gegeben, kurz verrührt und hiernach mittels Ultra Turrax bei 24.000 U/min homogenisiert. Die hieraus resultierende Präemulsion wurde mittels Hochdruckhomogenisators in 2 - 4 Zyklen, Druck 700 bar, feinstdispergiert. Die entstandene Dispersion wurde unter gleichmäßigem Rühren auf 30 °C abgekühlt.

Die Phasen 3 und 4 wurden jeweils in einem separaten Gefäß unter gleichmäßigem Rühren auf 50 °C erwärmt. Anschließend wurde die Phase 4 zur Phase 3 gegeben und hiernach per Ultra Turrax homogenisiert (12.000 U/min). Unter leichtem Rühren wurde die entstandene Dispersion auf 30 °C abgekühlt. Danach wurde die hochviskose Dispersion aus den Phasen 1 und 2 zugegeben. Anschließend wurde die Mischung bei 30 °C per Ultra Turrax (12.000 U/min) so lange homogenisiert, bis eine gleichmäßige Struktur vorlag. Die so erstellte Endformulierung konnte direkt verwendet werden.

### Ausführungsbeispiel I

### Herstellung eines Konzentrates mit dem Wirkstoff Octocrylene

| Inhaltsstoffe der Phase 1: | |
|---|---|
| 6,00 | Gew.% hydriertes Phosphatidylcholin |
| 20,00 | Gew.% Octocrylene |
| 1,00 | Gew.% Squalane |
| | |

| Inhaltsstoffe der Phase 2: | |
|---|---|
| 4,00 | Gew.% Glycerin |
| 5,00 | Gew.% Pentylene Glycol |
| ad 100,0 | Gew.% Wasser |

Die Inhaltsstoffe der Phase 1 wurden unter gleichmäßigem Rühren auf 80 °C erwärmt bis alle Inhaltsstoffe gelöst vorlagen. Ebenfalls wurde die Phase 2 in einem getrennten Gefäß auf 80 °C unter Rühren erwärmt. Anschließend wurde die Phase 2 zu der Phase 1 gegeben, kurz verrührt und hiernach mittels Ultra Turrax bei 15.000 U/min homogenisiert. Die hieraus resultierende Präemulsion wurde mittels Hochdruckhomogenisators in 5 - 6 Zyklen, Druck 800 bar, feinstdispergiert. Die entstandene Dispersion wird unter gleichmäßigem Rühren auf 30 °C abgekühlt.

Das so hergestellte Konzentrat kann durch entsprechende Verdünnung, vorzugsweise mit Wasser, einem Hydrokolloid und/oder Alkoholen, leicht in eine anwendungsfertige Endformulierung umgewandelt werden, wobei diese Endformulierung als Lichtschutzmittel bzw. als Sonnenschutzmittel verwendet wird.

### Ausführungsbeispiel J

### Herstellung einer als Lichtschutzmittel zu verwendenden Endformulierung mit dem Wirkstoff Octocrylene

| Inhaltsstoffe der Phase 1: | |
|---|---|
| 1,50 | Gew.% hydriertes Phosphatidylcholin |
| 5,00 | Gew.% Octocrylene |
| 0,25 | Gew.% Squalane |
| | |

| Inhaltsstoffe der Phase 2: | |
|---|---|
| 1,00 | Gew.% Glycerin |
| 1,25 | Gew.% Pentylene Glycol |
| 16,00 | Gew.% Wasser |
| | |

| Inhaltsstoffe der Phase 3: | |
|---|---|
| 15,00 | Gew.% C12-15 Alkyl Benzoate |
| 8,00 | Gew.% Titanium Dioxide |
| 0,10 | Gew.% Carbomer |
| 0,10 | Gew.% Sodium Carbomer |
| 0,10 | Gew.% Xanthan Gum |
| | |

| Inhaltsstoffe der Phase 4: | |
|---|---|
| 3,90 | Gew.% Pentylene Glycol |
| ad 100,0 | Gew.% Wasser |

Die Inhaltsstoffe der Phase 1 wurden unter gleichmäßigem Rühren auf 80 °C erwärmt bis alle Bestandteile gelöst vorlagen. Ebenfalls wurde die Phase 2 in einem getrennten Gefäß auf 80 °C unter Rühren erwärmt. Anschließend wurde die Phase 2 zu der Phase 1 gegeben, kurz verrührt und hiernach mittels Ultra Turrax bei 15.000 U/min homogenisiert. Die hieraus resultierende Präemulsion wurde mittels Hochdruckhomogenisators in 5 - 6 Zyklen, Druck 800 bar, feinstdispergiert. Die entstandene Dispersion wird unter gleichmäßigem Rühren auf 30 °C abgekühlt.

Die Phasen 3 und 4 wurden jeweils in einem separaten Gefäß unter gleichmäßigem Rühren auf 30 °C erwärmt. Anschließend wurde Phase 4 zur Phase 3 gegeben und hiernach per Ultra Turrax homogenisiert (10.000 U/min). Unter leichtem Rühren wurde die entstandene Dispersion auf 30 °C abgekühlt. Dann wurde die hochviskose Dispersion aus den Phasen 1 und 2 zugegeben. Danach wurde die Mischung bei 30 °C per Ultra Turrax (12.000 U/min) so lange homogenisiert, bis eine gleichmäßige Struktur vorlag. Die so erstellte Endformulierung konnte direkt verwendet werden.

### Vergleichsbeispiel A

Um eine vergleichende Ermittlung des Lichtschutzfaktors (LSF) durchführen zu können, wurde eine konventionelle Zusammensetzung erstellt, die denselben Wirkstoff in derselben Konzentration aufwies, wie dies zuvor im Ausführungsbeispiel J beschrieben ist. Hierbei wies die konventionelle Zusammensetzung folgende Inhaltsstoffe auf:

| Inhaltsstoffe der Phase 1: | |
|---|---|
| 1,50 | Gew.% PEG-20 Stearate |
| 5,00 | Gew.% Octocrylene |
| 0,25 | Gew.% Squalane |
| 1,00 | Gew.% Glycerin |
| 15,00 | Gew.% C12-15 Alkyl Benzoate |
| 8,00 | Gew.% Titanium Dioxide |
| 0,10 | Gew.% Carbomer |
| 0,10 | Gew.% Sodium Carbomer |
| | |

| Inhaltsstoffe der Phase 2: | |
|---|---|
| 0,10 | Gew.% Xanthan Gum |
| 4,15 | Gew.% Pentylene Glycol |
| ad 100,0 | Gew.% Wasser |

Die Inhaltsstoffe der Phase 1 wurden unter gleichmäßigem Rühren auf 80 °C erwärmt bis alle Wirkstoffe gelöst vorlagen. Ebenfalls wurde die Phase 2 in einem getrennten Gefäß auf 80 °C unter Rühren erwärmt. Anschließend wurde die Phase 2 zu der Phase 1 gegeben, kurz verrührt und hiernach mittels Ultra Turrax bei 15.000 U/min homogenisiert. Unter leichtem Rühren wurde die entstandene Dispersion auf 30°C abgekühlt. Danach wurde die Mischung bei 30 °C per Ultra Turrax (15000 U/min) so lange homogenisiert, bis eine gleichmäßige Struktur vorlag.

### Ausführungsbeispiel K

### Herstellung einer Endformulierung zur Anwendung gegen Zecken mit dem Wirkstoff Icaridin

| Inhaltsstoffe der Phase 1: | |
|---|---|
| 3,00 | Gew.% hydriertes Phosphatidylcholin |
| 10,00 | Gew.% Icaridin |
| | |

| Inhaltsstoffe der Phase 2: | |
|---|---|
| 1,80 | Gew.% Pentylene Glycol |
| 19,00 | Gew.% Wasser |
| | |

| Inhaltsstoffe der Phase 3: | |
|---|---|
| 5,00 | Gew.% Caprylic/ Capric Triglyceride |
| 0,10 | Gew.% Carbomer |
| 0,10 | Gew.% Sodium Carbomer |
| 0,10 | Gew.% Dehydroxanthan Gum |
| | |

| Inhaltsstoffe der Phase 4: | |
|---|---|
| 3,50 | Gew.% Pentylene Glycol |
| ad 100,0 | Gew.% Wasser |

Die Inhaltsstoffe der Phase 1 wurden unter gleichmäßigem Rühren auf 80 °C erwärmt bis alle Inhaltsstoffe gelöst vorlagen. Ebenfalls wurde die Phase 2 in einem getrennten Gefäß auf 80 °C unter Rühren erwärmt. Anschließend wurde die Phase 2 zu der Phase 1 gegeben, kurz verrührt und hiernach mittels Ultra Turrax bei 18.000 U/min homogenisiert. Die hieraus resultierende Präemulsion wurde mittels Hochdruckhomogenisators in 2 - 3 Zyklen, Druck 600 bar, feinstdispergiert. Die entstandene Dispersion wurde unter gleichmäßigem Rühren auf 30 °C abgekühlt.

Die Phasen 3 und 4 wurden jeweils in einem separaten Gefäß unter gleichmäßigem Rühren auf 30 °C erwärmt. Anschließend wurde die Phase 4 zur Phase 3 gegeben und per Ultra Turrax homogenisiert (12.000 U/mim). Unter leichtem Rühren wurde die entstandene Dispersion auf 30 °C abgekühlt. Danach wurde die hochviskose Dispersion aus den Phasen 1 und 2 zugegeben. Anschließend wurde die Mischung bei 30 °C per Ultra Turrax (11.000 U/min) so lange homogenisiert, bis eine gleichmäßige Struktur vorlag. Die so erstellte Endformulierung konnte direkt verwendet werden.

### Vergleichsbeispiel B

Um eine vergleichende Ermittlung der Wirksamkeit der zuvor beschriebenen Zusammensetzung gemäß Ausführungsbeispiel K gegen Zecken durchführen zu können, wurde eine konventionelle Zusammensetzung erstellt, die, wie Beispiel K, denselben Wirkstoff in derselben Konzentration aufwies.

| Inhaltsstoffe der Phase 1: | |
|---|---|
| 3,00 | Gew.% Polyglyceryl-3 Polyricenoleat |
| 10,00 | Gew.% Icaridin |
| 5,00 | Gew.% Caprylic/ Capric Triglyceride |
| 0,10 | Gew.% Carbomer |
| 0,10 | Gew.% Sodium Carbomer |
| 0,10 | Gew.% Dehydroxanthan Gum |
| | |

| Inhaltsstoffe der Phase 2: | |
|---|---|
| 5,00 | Gew.% Pentylene Glycol |
| ad 100,0 | Gew.% Wasser |

Zur Herstellung dieser konventionellen cremeartigen Zusammensetzung wurde die Inhaltsstoffe der Phase 1 unter gleichmäßigem Rühren auf 80 °C erwärmt bis alle Inhaltsstoffe gelöst vorlagen. Ebenfalls wurde die Phase 2 in einem getrennten Gefäß auf 80 °C unter Rühren erwärmt. Anschließend wurde die Phase 2 zu der Phase 1 gegeben, kurz verrührt und hiernach mittels Ultra Turrax bei 18.000 U/min homogenisiert. Unter leichtem Rühren wurde die entstandene Dispersion auf 30 °C abgekühlt. Danach wurde die Mischung bei 30 °C per Ultra Turrax (12.000 U/min) so lange homogenisiert, bis eine gleichmäßige cremeartige Struktur vorlag.

### Ausführungsbeispiel L

### Herstellung einer Endformulierung zur Anwendung gegen Läuse mit dem Wirkstoff Permethrin

| Inhaltsstoffe der Phase 1: | |
|---|---|
| 2,00 | Gew.% hydriertes Phosphatidylcholin |
| 0,40 | Gew.% Permethrin |

| Inhaltsstoffe der Phase 2: | |
|---|---|
| 1,80 | Gew.% Pentylene Glycol |
| 19,00 | Gew.% Wasser |
| | |

| Inhaltsstoffe der Phase 3: | |
|---|---|
| 5,00 | Gew.% Caprylic/ Capric Triglyceride |
| 0,06 | Gew.% Carbomer |
| 0,06 | Gew.% Sodium Carbomer |
| 0,05 | Gew.% Dehydroxanthan Gum |
| | |

| Inhaltsstoffe der Phase 4: | |
|---|---|
| 3,50 | Gew.% Pentylene Glycol |
| ad 100,0 | Gew.% Wasser |

Die Inhaltsstoffe der Phase 1 wurden unter gleichmäßigem Rühren auf 80 °C erwärmt bis alle Wirkstoffe gelöst vorlagen. Ebenfalls wurde die Phase 2 in einem getrennten Gefäß auf 80 °C unter Rühren erwärmt. Anschließend wurde die Phase 2 zu der Phase 1 gegeben, kurz verrührt und hiernach mittels Ultra Turrax bei 9.000 U/min homogenisiert. Die hieraus resultierende Präemulsion wurde mittels Hochdruckhomogenisators in 3 - 5 Zyklen, Druck 800 bar, feinstdispergiert. Die entstandene Dispersion wurde unter gleichmäßigem Rühren auf 30 °C abgekühlt.

Die Phasen 3 und 4 wurden jeweils in einem separaten Gefäß unter gleichmäßigem Rühren auf 30 °C erwärmt. Anschließend wurde die Phase 4 zur Phase 3 gegeben und per Ultra Turrax homogenisiert (9.000 U/min). Unter leichtem Rühren wurde die entstandene Dispersion auf 30 °C abgekühlt. Danach wurde die hochviskose Dispersion aus den Phasen 1 und 2 zugegeben. Hiernach wurde die Mischung bei 30 °C per Ultra Turrax (9.000 U/min) so lange homogenisiert, bis eine gleichmäßige Struktur vorlag. Die so erstellte Endformulierung konnte direkt verwendet werden.

### Ausführungsbeispiel M

### Herstellung einer Endformulierung zur Anwendung bei Rosacea mit dem Wirkstoff Vitamin K

| Inhaltsstoffe der Phase 1: | |
|---|---|
| 1,50 | Gew.% hydriertes Phosphatidylcholin |
| 5,00 | Gew.% Vitamin K |
| 0,20 | Gew.% Squalane |
| 0,10 | Gew.% Rice bran Wax |
| 1,00 | Gew.% Caprylic / Capric Triglyceride |
| 0,25 | Gew.% Phenylethylacohol |
| | |

| Inhaltsstoffe der Phase 2: | |
|---|---|
| 18,00 | Gew. % Wasser |
| | |

| Inhaltsstoffe der Phase 3: | |
|---|---|
| 5,00 | Gew.% Caprylic/ Capric Triglyceride |
| 0,10 | Gew.% Carbomer |
| 0,10 | Gew.% Sodium Carbomer |
| 0,20 | Gew.% Hydroethylcellulose |
| | |

| Inhaltsstoffe der Phase 4: | |
|---|---|
| 3,50 | Gew.% Pentylene Glycol |
| ad 100,0 | Gew.% Wasser |

Die Inhaltsstoffe der Phase 1 wurden unter gleichmäßigem Rühren auf 75 °C erwärmt bis alle Inhaltsstoffe gelöst vorlagen. Ebenfalls wurde die Phase 2 in einem getrennten Gefäß auf 75 °C unter Rühren erwärmt. Anschließend wurde die Phase 2 zu der Phase 1 gegeben, kurz verrührt und hiernach mittels Ultra Turrax bei 15.000 U/min homogenisiert. Die hieraus resultierende Präemulsion wurde mittels Hochdruckhomogenisators in 3 - 4 Zyklen, Druck 800 bar, feinstdispergiert. Die entstandene Dispersion wurde unter gleichmäßigem Rühren auf 30 °C abgekühlt.

Die Phasen 3 und 4 wurden jeweils in einem separaten Gefäß unter gleichmäßigem Rühren auf 30 °C erwärmt. Anschließend wurde die Phase 4 zur Phase 3 gegeben und hiernach per Ultra Turrax homogenisiert (12.000 U/min). Unter leichtem Rühren wurde die entstandene Dispersion auf 30 °C abgekühlt. Danach wurde die hochviskose Dispersion aus den Phasen 1 und 2 zugegeben. Anschließend wurde die Mischung bei 30 °C per Ultra Turrax (10.000 U/min) so lange homogenisiert, bis eine gleichmäßige Struktur vorlag. Die so erstellte Endformulierung konnte direkt verwendet werden.

### Nachweis der Wirksamkeit der in dem Ausführungsbeispiel G beschriebenen Endformulierung bei Herpesbefall

10 Probanden (6 weibliche, 4 männliche) in einem Alter zwischen 25 Jahren und 55 Jahren, die alle seit mindestens zwei Jahren an unregelmäßig auftretenden Herpesinfektionen, insbesondere im Bereich der Lippen und unterhalb der Nase, litten, wurden zum Höhepunkt der Herpesinfektion mit der Endformulierung gemäß Ausführungsbeispiel G behandelt. Alle Probanden beklagten sich einerseits über einen starken Juckreiz und andererseits über Schmerzen.

In einer ersten Versuchsreihe wurden die Probanden mit einer herkömmlichen Salbe, die den Wirkstoff Hypericin in der mit dem Ausführungsbeispiel G entsprechenden Konzentration enthielt, behandelt. Die Anzahl der täglichen Applikationen der herkömmlichen Salbe war den Probanden selbst überlassen.

Vor Beginn der Applikation, nach zwei Tagen, nach vier Tagen und nach acht Tagen wurde durch eine subjektive Beurteilung der Grad des Herpes-Befalls und der damit verbundenen Begleiterscheinungen erfaßt und benotet. Hierfür wurde folgende Benotung zugrundegelegt:
0 = kein erkennbarer Herpesbefall
1 = soeben noch erkennbarer Herpesbefall
2 = leichter Herpesbefall
3 = mittlerer Herpesbefall
4 = starker Herpesbefall
5 = sehr starker Herpesbefall

Desweiteren wurde die Zeit bis zur Abheilung des akuten Herpesbefalls in Tagen bestimmt, wobei hierfür mindestens eine Note 1 vergeben werden mußte.

Das Ergebnis dieser ersten Versuchsreihe ist in der nachfolgenden Tabelle wiedergegeben.

**herkömmliche, Hypericin-haltige Salbe**

| Proband-Nr./Geschlecht | vor Beginn | nach 2 Tagen | nach 4 Tagen | nach 8 Tagen | Abheilung nach Tagen |
|---|---|---|---|---|---|
| 1 / m | 5 | 5 | 4 | 3 | 20 |
| 2 / m | 5 | 4 | 3 | 2 | 17 |
| 3 / m | 5 | 5 | 4 | 3 | 24 |
| 4 / m | 4 | 4 | 3 | 2 | 14 |
| 5 / w | 4 | 4 | 3 | 2 | 8 |
| 6 / w | 3 | 2 | 3 | 2 | 7 |
| 7 / w | 5 | 5 | 4 | 2 | 10 |
| 8 / w | 4 | 4 | 3 | 2 | 7 |
| 9 / w | 5 | 4 | 3 | 1 | 5 |
| 10 / w | 5 | 4 | 4 | 3 | 21 |

Die zuvor ausgewählten Probanden wurden für eine zweite Versuchsreihe dann eingestellt, wenn sie wieder an einem akuten Herpesbefall litten. Hierbei variierte die Zeit zwischen der ersten Versuchsreihe und der zweiten Versuchsreihe je nach Proband zwischen drei Monaten und neun Monaten.

In der zweiten Versuchsreihe wurden die Probanden mit der im Ausführungsbeispiel G spezifizierten Zusammensetzung behandelt, wobei es den Probanden selbst überlassen war, die Anzahl der täglichen Applikationen der Zusammensetzung gemäß Ausführungsbeispiel G zu bestimmen.

Die Auswertung dieser zweiten Versuchsreihe erfolgte analog zur Auswertung der ersten Versuchsreihe und ist in der nachfolgenden Tabelle wiedergegeben. Hierzu ist anzumerken, daß die Probanden-Nr. der ersten Versuchsreihe mit der Probanden-Nr. der zweiten Versuchsreihe identisch ist.

**Zusammensetzung gemäß Ausführungsbeispiel G**

| Proband-Nr./Geschlecht | vor Beginn | nach 2 Tagen | nach 4 Tagen | nach 8 Tagen | Abheilung nach Tagen |
|---|---|---|---|---|---|
| 1 / m | 5 | 4 | 3 | 1 | 10 |
| 2 / m | 5 | 3 | 2 | 1 | 8 |
| 3 / m | 4 | 3 | 3 | 1 | 8 |
| 4 / m | 5 | 3 | 2 | 1 | 6 |
| 5 / w | 4 | 3 | 2 | 1 | 5 |
| 6 / w | 4 | 3 | 2 | 0 | 0 |
| 7 / w | 5 | 4 | 3 | 1 | 3 |
| 8 / w | 5 | 4 | 2 | 0 | 0 |
| 9 / w | 4 | 3 | 2 | 0 | 0 |
| 10 / w | 4 | 2 | 2 | 1 | 2 |

Der Vergleich der zuvor wiedergegebenen beiden Tabellen belegt eindeutig die Überlegenheit der Zusammensetzung gemäß Ausführungsbeispiel G im Vergleich zu der herkömmlichen Salbe. Insbesondere berichteten alle Probanden übereinstimmend, daß insbesondere bereits nach wenigen Applikationen der Juckreiz und der Schmerz deutlich nachließen, was bei der herkömmlichen Salbe so nicht der Fall war.

### Bestimmung des Lichtschutzfaktors der Zusammensetzung gemäß Ausführungsbeispiel J

Für die Bestimmung des Lichtschutzfaktors wurde die COLIPA-Lichtschutzfaktor-Prüfmethode herangezogen. Diese Methode ist eine Labormethode, die eine künstliche, Ultraviolett (UV)- Lichtquelle mit definierter, bekannter Leistung voraussetzt. Bei der Durchführung wird eine abgestufte Serie verzögerter UV-Erythemreaktionen auf mehreren kleinen Hautarealen ausgewählter Probanden induziert.

Die Probanden müssen mindestens zweimal im Prüflaboratorium vorstellig werden: Beim ersten Mal werden sie den erforderlichen UV-Dosen ausgesetzt, beim zweiten Mal wird die durch Sonnenschutzprodukte hervorgerufene Verzögerung der Erythemreaktionen bei identischem Versuchsaufbau beurteilt. Durch die stufenweise Steigerung der UV-Dosis werden unterschiedliche Hauterythemgrade (Rötung als Folge einer oberflächlichen Vasodilation) erzeugt, die etwa 24 Stunden nach der UV-Exposition einen Maximalwert erreichen. Die Expositionszeit, die an ungeschützter Haut Typ II und III nach Fitzpatrick ein Erythem bewirkt, beträgt in der Regel etwa zwei Minuten. Die niedrigste Dosis, die einen eindeutigen Erythembereich erzeugt, ist die minimale Erythemdosis oder MED. Die MED für ungeschützte Haut (MEDu; u steht für "unprotected") und die MED nach Auftragung eines Sonnenschutzmittels (d.h. die MED für geschützte Haut = MEDp; p steht für "protected") werden gleichzeitig am selben Probanden ermittelt. Die MEDu und die MEDp können visuell von geschulten Bewertern oder instrumentell mit einem Colorimeter bewertet werden. Dabei können mehrere Präparate am selben Probanden gleichzeitig geprüft werden. Der Lichtschutzfaktor des Präparates wird für jeden Probanden auf der Grundlage des Verhältnisses der MEDp zur MEDu errechnet. Ein Präparat muß an mindestens zehn Probanden geprüft werden. Die Vertrauensgrenzen für den mittleren Lichtschutzfaktor sollten +/- 20% innerhalb des Mittelwertes liegen, d.h. wenn der mittlere Lichtschutzfaktor 10 beträgt, sollten die berechneten Vertrauensgrenzen oberhalb 8 bzw. unterhalb von 12 liegen. Falls dies nicht der Fall ist, müssen Prüfungen an weiteren Probanden durchgeführt werden, bis die statistischen Kriterien erfüllt oder 20 Probanden eingesetzt worden sind. Der mittlere Lichtschutzfaktor eines Präparates wird aus den Ergebnissen aller Probanden errechnet.

Die Lichtschutzfaktor-Prüfmethode von COLIPA beschreibt weiterhin ein standardisiertes Verfahren für die Auftragung und Verteilung der Sonnenschutzmittel auf den Prüfflächen, da diese Phase der Prüfung als eine wesentliche Quelle für experimentelle Fehler identifiziert wurde. Bei allen Prüfungen sollte entsprechend dem erwarteten Lichtschutzfaktor der Prüfrezepturen ein Standardpräparat nach COLIPA mit entsprechend hohem oder niedrigem Lichtschutzfaktor eingesetzt werden.

Bei den Probanden erfolgt eine Untersuchung des Testareals von der unteren Linie der Schulterblätter bis zur Taillenhöhe. Auf dem Rücken jedes Probanden werden Hinweise auf Sonnenbrand, Sonnenbräune, Narben, Hautläsionen und unregelmäßige Pigmentierung bestimmt. Wenn nach Ansicht des Untersuchers eines der aufgeführten Artefakte in signifikanter Weise vorhanden ist, wird der Proband aus der Studie ausgeschlossen. Die Untersuchung wurde an 20 Probanden durchgeführt.

### Klassifikation der Hauttypen nach Fitzpatrick

Die Hauttypen werden folgendermaßen eingeteilt:
*Hauttyp I* Bräunung : nie, Sonnenbrand: immer
*HauttypII* Bräunung: wenig, Sonnenbrand: immer
*Hauttyp III* Bräunung: mäßig, Sonnenbrand: selten

Als UV-Quelle wird im Solar Light Company's 601-300 Multiport Simulator das Spektrum einer Xenon-Bogenlampe durch besondere Filter auf den erythemal wirksamen Bereich abgebildet (COLIPA-Spektrum) und auf die Haut appliziert. Der Simulator ist mit 6 Bestrahlungsfeldern ausgestattet, die gleichzeitig unterschiedliche Bestrahlungsdosen emittieren können. Durch einen individuellen zeit- oder leistungsgesteuerten Schließmechanismus können verschiedene UV-Dosen verabreicht und so eine "Lichttreppe" bestimmt werden. Der LSF wird durch Messung eines Produktfeldes (z.B. Sonnencreme) und eines Leerfeldes (ungeschützte Haut) ermittelt. Die Bestrahlung kann durch einen drehbaren Strahlenausgang sowohl im Sitzen als auch im Liegen durchgeführt werden.

Um das angeborene Reaktionsvermögen jedes Probanden auf UV-Strahlung festzulegen wird eine Reihe mit 6 UV-Bestrahlungen 24 Stunden vor der eigentlichen Untersuchung vorgenommen. Jedes Bestrahlungsfeld ist 1 cm im Durchmesser. Die Zeitintervalle sind als geometrische Reihe gewählt, wobei die Bestrahlungsdauer mit jedem Feld um 25 % verlängert wird. Die bestrahlten Areale werden 16-24 Stunden nach UV-Exposition beurteilt und die MEDu (MED der ungeschützten Haut) bestimmt. Die MED (Minimalen Erythemdosis) dient als Indikator für die zu applizierende Dosis für die Lichtschutzfaktor-Untersuchung (LSF-Untersuchung). Die MED ist definiert als die Bestrahlungsenergie, die benötigt wird um eine schwache, jedoch eindeutig wahrnehmbare Hautrötung mit scharfer Begrenzung zu erzeugen. Die Bestrahlungsdosis in dieser Untersuchung wurde zeitlich erfaßt.

Der LSF für die Zusammensetzung gemäß Ausführungsbeispiel J wurde im Vergleich zu dem LSF der Zusammensetzung gemäß Vergleichsbeispiel A an eindeutigen Positionen auf den Rücken der Probanden (n = 20) bestimmt. Die Bestimmung der Positionen wurde wie folgt durchgeführt:
- Markierung des gesamten Testareals
- Markierung der einzelnen Testareale bei 35 cm², jeweils für die zuvor genannten beiden zu vergleichenden Ausführungsbeispielen.

Auf jedes Testfeld wird die jeweilige Zusammensetzung (Ausführungsbeispiel J oder Vergleichsbeispiel A) mit einer Menge von 2 mg/cm² ± 0,02 aufgetragen. Nach der Applikation wird ein Intervall von etwa 15 Min. als Einwirkzeit vor der UV-Bestrahlung abgewartet.

Nach Ablauf der Einwirkzeit wird zunächst ein ungeschütztes Areal auf dem Rücken des Probanden bestrahlt. Anschließend wird der Test auf den mit der jeweiligen Zusammensetzung behandelten Arealen wiederholt.

Der gleiche Test wird auf einem zweiten Testareal 2 h nach Applikation des Produktes wiederholt. Die Testfelder werden mit einer Folge von UV-Bestrahlungseinheiten verschiedener Intensität behandelt. Die tatsächliche Expositionszeit wird anhand der zuvor bestimmten MED der Versuchsperson und des vermuteten LSF des Produktes gewählt. Genauer, die MED wird mit dem vermuteten LSF des Produktes multipliziert, hieraus resultiert die Bestrahlungszeit. Es wird als UV-Dosis eine 25 % geometrische Reihe ausgewählt. Nach Abschluß der Bestrahlung wird die Position der Testfelder markiert. Jeder Proband wird zum Schutz vor weiterer UV-Strahlung aufgefordert, das gesamte Testareal abzudecken.

Die Bewertung der behandelten und bestrahlten Testfelder wurde 20-24 Stunden nach UV-Exposition durch geschultes Personal vorgenommen.

Die individuellen und gemittelten LSF-Werte für die Zusammensetzung gemäß Ausführungsbeispiel J und die Zusammensetzung gemäß Vergleichsbeispiel A sind in der folgenden Tabelle angegeben.

| Beispiel Sonne | | | |
|---|---|---|---|
| untersuchte Probe | Bestrahlun g nach Einwirkzeit von | Mittelwert des LSF | Standardabweichung |
| Ausführungsbeispiel J | 15 min. | 23,6 | 2,3 |
| Ausführungsbeispiel J | 120 min. | 21,2 | 4,6 |
| Vergleichsbeispiel A | 15 min. | 13,7 | 4,5 |
| Vergleichsbeispiel A | 120 min. | 9,8 | 5,9 |

Im Zusammenhang mit der durchgeführten Messung ist noch zu erwähnen, daß der hohe Lichtschutzfaktor der Zusammensetzung gemäß Ausführungsbeispiel J selbst nach einer Einwirkzeit von 120 Minuten noch vorhanden war, was bei der Zusammensetzung gemäß Vergleichsbeispiel A nicht der Fall war. Hieraus ist zu schließen, daß aufgrund der speziellen Struktur der Zusammensetzung gemäß Ausführungsbeispiel J diese stabil im Stratum corneum positioniert ist, was bei der Zusammensetzung gemäß Vergleichsbeispiel A nicht der Fall war. Hier fiel der LSF drastisch von 13,7 auf 9,8 ab.

Nachweis der Wirksamkeit der in dem Ausführungsbeispiel K beschriebenen Endformulierung zur Verhinderung der Zeckenkontamination

Um die Wirksamkeit der Zusammensetzung gemäß Ausführungsbeispiel K im Vergleich zu der konventionellen Zusammensetzung gemäß Vergleichsbeispiel B zu überprüfen, wurde ein lebendes, narkotisiertes Hausschwein der Gattung Sus Scrofa Domestica (Alter 2 ½ Jahre) jeweils auf seiner linken Seite und seiner rechten Seite vollständig rasiert. Die beiden Schweineseiten wurden entlang der Wirbelsäule durch einen 3 cm breiten doppelt klebenden Klebestreifen voneinander abgegrenzt, um so eine Wanderung der Zecken von der einen Schweineseite auf die andere Schweineseite zu verhindern. Die übrigen Ränder der Schweineseiten wurden ebenfalls mit diesem Klebestreifen versehen.

Nach Lagefixierung des betäubten Schweines in aufrechter Stellung wurde auf die eine Schweineseite (Meßfläche etwa 500 cm²) die Zusammensetzung gemäß Ausführungsbeispiel K und auf die andere Schweineseite (Meßfläche etwa 500 cm²) die Zusammensetzung gemäß Vergleichsbeispiel B jeweils in einer Konzentration von 1 g/10 cm² aufgetragen und gleichmäßig über die Meßflächen verrieben. Nach einer Einwirkzeit von 10 Minuten wurde jede Schweineseite mit einer Zeckenpopulation von gleichem Entwicklungsstadium und von gleicher Zeckenanzahl (jeweils 20 Zecken) besiedelt.

Die Anzahl der Zecken wurde nach einem Zeitraum von vier Stunden nach Besiedelung auf jeder Schweineseite festgestellt. Hierbei wurde unterschieden, wieviel Zecken sich festgebissen hatten und wieviel Zecken ohne Biß die jeweilige Schweineseite noch besiedelte. Ferner wurden die abgewanderten Zecken, die im Klebestreifen fixiert waren, gezählt. Desweiteren wurde mikroskopisch überprüft, ob die Zecken nach vier Stunden noch lebten.

Die Ergebnisse dieser Untersuchung sind in der nachfolgenden Tabelle wiedergegeben:

| | Zusammensetzung gemäß Ausführungsbeispiel K | Zusammensetzung gemäß Vergleichsbeispiel B |
|---|---|---|
| Ausgangs-Zeckenanzahl | 20 | 20 |
| Zeckenanzahl ohne Biß | 6 | 1 |
| Zeckenanzahl mit Biß | 8 | 16 |
| abgewanderte Zecken | 6 | 3 |
| tote Zecken (insgesamt) | 16 | 8 |

Der Vergleich der zuvor wiedergegebenen Tabellen belegt eindeutig die Überlegenheit der Zusammensetzung gemäß Ausführungsbeispiel K im Vergleich zu der herkömmlichen Zusammensetzung gemäß Vergleichsbeispiel B. Insbesondere die Tatsache, daß sich nur acht Zecken in der Haut verankert haben und daß eine wesentlich höhere Anzahl von toten Zecken festgestellt werden konnten, belegen, daß die Zusammensetzung gemäß Ausführungsbeispiel K hochwirksam ist.

Zu den bei den Ausführungsbeispielen A bis M eingesetzten hydrierten Phosphatidylcholinen ist festzuhalten, daß diese eine Konzentration an hydriertem Phosphatidylcholin von 93 ± 3 Gew.% aufweist und daß die Acylreste zu 85 Gew.% aus Stearinsäure und zu 14 Gew.% aus Palmitinsäure bestehen.

## Patentansprüche

1. Topisch zu applizierende kosmetische oder pharmazeutische Zusammensetzung, die eine hydrophile äußere Phase, mindestens einen kosmetischen und/oder pharmazeutischen Wirkstoff sowie mindestens eine Trägersubstanz für den Wirkstoff aufweist und die Trägersubstanz solche Strukturen ausbildet, die mindestens zwei, sandwichartig übereinander angeordnete lamellare Doppelmembranschichten umfaßt, wobei zwischen benachbarten parallel zueinander ausgerichteten Doppelmembranschichten jeweils eine Schicht einer inneren Phase angeordnet ist, **dadurch gekennzeichnet, daß** der Wirkstoff in der Doppelmembranschicht und in der Schicht der inneren Phase verteilt ist, derart, daß die Schicht der inneren Phase den Wirkstoff in einem Konzentrationsbereich zwischen 2 Gew.% und 98 Gew.% und die Doppelmembranschicht den Wirkstoff in einer Konzentration zwischen 98 Gew.% und 2 Gew.%, jeweils bezogen auf die Gesamtkonzentration an Wirkstoff, enthalten, und daß die äußere Phase keinen oder nahezu keinen Wirkstoff aufweist.

2. Zusammensetzung nach Anspruch 1, welcher mindestens einen kosmetischen Wirkstoff umfaßt, der ausgewählt ist aus der Gruppe die Reiskeimwachs, Mono-, Di-, Tri- und/oder Polyglyceride der Rizinolsäure, der 12-Hydroxystearinsäure und/oder der 11-Hydroxypalmitinsäure, Rizinolsäureoctyldedecylester, 12-Hydroxystearinsäureoctylester, Bienenwachs, Japanwachs, Carnaubawachs, Cetylpalmitat, Kakaobutter, Sheabutter, Squalan, Cholesterin, Cholesterylsulfat, Phytosterole und/oder Lanolin umfaßt.

3. Kosmetische Zusammensetzung nach Anspruch 2, **dadurch gekennzeichnet, dass** die hydrophile äußere Phase Wasser enthält, die Schicht der inneren Phase Wasser enthält, die äußere Phase eine Wirkstoffkonzentration zwischen 0 Gew.% und 2 Gew.%, bezogen auf die Gesamtkonzentration des Wirkstoffes in der Zusammensetzung, aufweist, und die Trägersubstanz hydriertes Phosphatidylcholin aufweist.

4. Zusammensetzung nach einem der vorangehenden Ansprüche, **dadurch gekennzeichnet, daß** der Wirkstoff zu mindestens 70 Gew.%, bezogen auf die Gesamtkonzentration an Wirkstoff, innerhalb der Doppelmembranschicht angeordnet ist.

5. Zusammensetzung nach Anspruch 4, **dadurch gekennzeichnet, daß** innerhalb der Doppelmembranschicht der hydrophobe Wirkstoff in einer Konzentration zwischen 80 Gew.% und 90 Gew.%, bezogen auf die Gesamtkonzentration an Wirkstoff, eingelagert ist.

6. Zusammensetzung nach einem der vorangehenden Ansprüche, **dadurch gekennzeichnet, daß** die Zusammensetzung einen zweiten kosmetischen Wirkstoff umfaßt, der ein Lichtschutzfilter ist, der ausgewählt ist aus der Gruppe die PABA und Derivate davon, Octyl dimethyl PABA, Homosalate, Oxybenzon BEMT, p-Methoxycinnamat, Ethylhexyl Triazone, Octocrylen, Benzophenon-3, Benzophenon-4, Benzophenon-9, Diethylamino-hydroxybenzoylhexylbenzoat, Drometrizole Trisiloxan, 4-Methylbenzylidene Campher, 3-Benzylidene Campher, Octylsalicylat, Methylene Bis-Benzotriazolyl Tetramethylbutylphenol und Bis-Ethylhexyloxyphenol Methoxyphenyl Triazin, Ethylhexylmethoxycinnamate, Diethylhexyl Butamido Triazone, Phenylbenzimidazol Sulfonic Acid, Butyl Methoxydibenzoylmethane, Diethylamino Hydroxybenzoyl Hexyl Benzoate, Disodium Phenyl Dibenzimidazole Tetrasulfonate und Terephthalylidene Dicamphor Sulfonic Acid umfaßt.

7. Zusammensetzung nach einem der vorangehenden Ansprüche, **dadurch gekennzeichnet, daß** die Zusammensetzung einen zweiten kosmetischen Wirkstoff umfaßt, der ein Antioxidant ist.

8. Zusammensetzung nach einem der vorangehenden Ansprüche, **dadurch gekennzeichnet, daß** der Antioxidant ausgewählt ist aus der Gruppe die Vitamin A, und Vitamin C, Tocopherole, Carcinin, Liponsäure, Liposol Maleate, Carotenoide, Lycopene, farblose Carotenoide, Polyphenole, Epicatechine, Epigallocatechine, Epigallocatechingallat, Epicatechnin-3-gallate, Kaffeesäure, Kaffeesäureester, Rosmarinsäure, Flavonoide, Flavanole, Flavanone, Anthocyanidine, Proanthocyanidine, Resveratrol, Silymarin, Aspalathin, Ellaginsäure, Curcuminderivate, Dihydroquercetin, N.D.G.A., Rutin, Tetrahydrocurcuminoid, Tetrahydrodiferuloylmethane, Tetrahydrodemethoxydiferuloylmethane, Tetrahydrobisdemethoxydiferuloylmethane, Glutathione, Coenzyme q 10, L-Carnosin, N-Acetylcystein, Phytinsäure, Furalglucytol, Thioctinsäure, EDTA, BHA, BHT, SOD und 4-Thiazolidinonekinetin umfaßt.

9. Zusammensetzung nach einem der vorangehenden Ansprüche, **dadurch gekennzeichnet, daß** die Zusammensetzung als pflegende kosmetische Wirkstoffe Sheabutter, Ceramide, insbesondere Ceramide-3, Cupuacu-Butter, Squalan und/oder Triglyceride, insbesondere mittelkettige, gesättigte C₈-C₂₄-Triglyceride, enthält.

10. Zusammensetzung nach einem der vorangehenden Ansprüche, **dadurch gekennzeichnet, daß** die Zusammensetzung den Wirkstoff in einer Konzentration zwischen 0,01 Gew.% und 35 Gew.%, vorzugsweise zwischen 0,1 Gew.% und 15 Gew.%, bezogen auf die anwendungsfertige Zusammensetzung, aufweist.

11. Zusammensetzung nach einem der vorangehenden Ansprüche, **dadurch gekennzeichnet, daß** die Zusammensetzung die Trägersubstanz in einer Konzentration zwischen 0,5 Gew.% und 30 Gew.%, vorzugsweise in einer Konzentration zwischen 0,7 Gew.% und 10 Gew.%, bezogen auf die anwendungsfertige Zusammensetzung, aufweist.

12. Zusammensetzung nach einem der vorangehenden Ansprüche, **dadurch gekennzeichnet, daß** die Zusammensetzung desweiteren mindestens einen Alkohol, insbesondere einen mehrwertigen Alkohol, enthält.

13. Zusammensetzung nach Anspruch 12, **dadurch gekennzeichnet, daß** die Zusammensetzung als Alkohol Phenylethylalkohol, Pentylenglykol, Caprylyl Glykol, Decylen Glykol und/oder Glycerin aufweist.

14. Zusammensetzung nach einem der vorangehenden Ansprüche, **dadurch gekennzeichnet, daß** die Zusammensetzung ein N-Acyl-Ethanolamin in einer Konzentration zwischen 0,01 Gew.% und 10 Gew.%, vorzugsweise zwischen 0,1 Gew.% und 3 Gew.%, bezogen auf die anwendungsfertige Zusammensetzung, aufweist.

15. Zusammensetzung nach Anspruch 14, **dadurch gekennzeichnet, daß** der Acylrest des N-Acyl-Ethanolamins ein C₁-C₂₄-Acylrest ist und vorzugsweise das N-Acyl-Ethanolamin aus der Gruppe ausgewählt ist, die N-Acetyl-Ethanolamin, N-Oleoyl-Ethanolamin, N-Linolenoyl-Ethanolamin, N-Cocoyl-Ethanolamin und N-Palmitoyl-Ethanolamin umfaßt.

16. Zusammensetzung nach einem der vorangehenden Ansprüche, **dadurch gekennzeichnet, daß** die Zusammensetzung desweiteren mindestens ein Konservierungsmittel, ein Antioxidanz, ein Verdickungsmittel und/oder einen Gelbildner aufweist.

17. Zusammensetzung nach einem der vorangehenden Ansprüche, **dadurch gekennzeichnet, daß** die Zusammensetzung jeweils zwischen
0,5 Gew.% und 7 Gew.% hydriertes Phosphatidylcholin,
0,5 Gew.% und 10 Gew.% Cupuacu Butter
0,5 Gew.% und 15 Gew.% Sheabutter
0,001 Gew.% und 3 Gew.% Ceramide
0,1 Gew.% und 5 Gew.% des Kolloids oder Hydrokolloids
2 Gew.% und 42 Gew.% des Öls oder des Ölbestandteils
0,01 Gew.% und 5 Gew.% des Wirkstoffs
0 Gew.% und 10 Gew.% sonstiger Zusätze und
5 Gew.% und 96 Gew.% Wasser
aufweist.
